# EUROPEAN PATENT APPLICATION

(11) **EP 2 179 651 A1**
(43) Date of publication of application: **28.04.2010**
(21) Application number: 08790662.4
(22) Date of filing: 26.06.2008
(51) Int. Cl.: A01N 37/18, A01P 3/00, C07C 235/46, C07C 235/48, C07C 235/54

(54) **PLANT DISEASE CONTROL AGENT, AND PLANT DISEASE CONTROL METHOD**

(30) Priority: 29.06.2007 JP 2007171888; 29.06.2007 JP 2007171889
(71) Applicant: Sumitomo Chemical Company, Limited, Tokyo 104-8260 (JP)
(72) Inventor: KOMORI, Takashi, Nerima-ku Tokyo 176-0013 (JP); KUBOTA, Mayumi, Toyonaka-shi Osaka 561-0802 (JP); MATSUZAKI, Yuichi, Toyonaka-shi Osaka 560-0021 (JP)
(74) Representative: Vossius & Partner
(86) International application number: PCT/JP2008/061647
(87) International publication number: WO 2009/004978

(57) **Abstract**

Disclosed is a plant disease control agent comprising an amide compound represented by the formula (1) as an active ingredient.
(1): 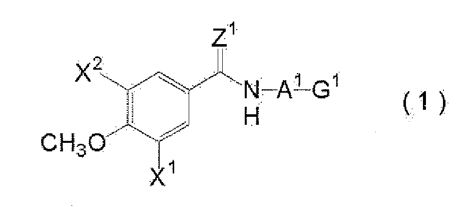 wherein X¹ represents a fluorine atom or a methoxy group; X² represents a hydrogen atom, a halogen atom, a C₁-C₄ alkyl group, a C₂-C₄ alkenyl group, a C₁-C₄ haloalkyl group, a C₁-C₄ alkoxy group, a C₁-C₄ alkylthio group, a C₁-C₄ hydroxyalkyl group and so on; Z¹ represents an oxygen atom or a sulfur atom; A¹ represents a single bond, -CH₂- and so on; and G¹ represents a group CR⁶R⁷R⁸, a C₃₋₆ cycloalkyl group and so on.

## Description

### Technical Field

The present invention relates to a plant disease control agent and a plant disease control method.

### Background Art

Heretofore, various plant disease control agents have been developed and put into practice (see, for example, Non-Patent Document 1). However, these plant disease control agents do not necessarily exhibit sufficient control activity.
As a certain amide compound, for example, N-(cyclohexylmethyl)-3-fluoro-4-methoxybenzamide (CAS No. 930863-91-5) is known.

Non-Patent Document 1: Narumi Shibuya, Hiroshi Kawahata, Mariko Kawahata, and Isao Shimazaki ed., "SHIBUYA INDEX"-2005-(10th Edition), SHIBUYA INDEX RESEARCH GROUP

### Disclosure of the Invention

### Problems to be Solved by the Invention

An object of the present invention is to provide an excellent plant disease control agent.

### Means for Solving the problems

The present inventors have studied in order to find out an excellent plant disease control agent and, as a result, have found that a plant disease control agent containing an amide compound represented by the following formula (1) as an active ingredient has excellent control acitivity. Thus, the present invention has been completed.
The present invention provides [1] to [21] shown below.

[1] A plant disease control agent comprising, as an active ingredient, an amide compound represented by the formula (1): 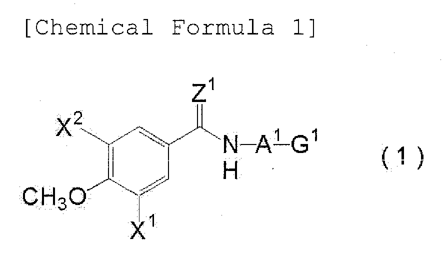 wherein
X¹ represents a fluorine atom or a methoxy group,
X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group or a CONR⁴R⁵ group, or a phenyl group optionally substituted with at least one member selected from the group consisting of a methyl group, a halogen atom, a cyano group and a nitro group,
Z¹ represents an oxygen atom or a sulfur atom,
A¹ represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G¹ represents a CR⁶R⁷R⁸ group (provided that the aforementioned A¹ is not a single bond when G¹ is a CR⁶R⁷R⁸ group), a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ represents a C1-C4 alkyl group, a C3-C4 alkenyl group or a C3-C4 alkynyl group,
R⁴ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a hydrogen atom, a C1-C4 alkyl group, a C3-C4 alkenyl group, a C3-C4 alkynyl group or a C2-C4 haloalkyl group,
R⁶ and R⁷ independently represent a C1-C4 alkyl group,
R⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR⁹R¹⁰ group, in which R⁹ and R¹⁰ are as defined above.

[2] The plant disease control agent according to [1], wherein in the formula (1),
A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming ring is replaced by a carbonyl group, or a C3-C6 hydroxyiminocycloalkyl group optionally substituted with at least one member selected from the group [a-1].

[3] The plant disease control agent according to [2], wherein in the formula (1),
X² is a hydrogen atom or a halogen atom, and
G¹ is a C3-C6 cycloalkyl group optionally substituted with at least one member selected from the group consisting of a halogen atom, a C1-C4 alkyl group and a hydroxyl group.

[4] The plant disease control agent according to [1], wherein in the formula (1),
A¹ is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a CR⁶R⁷R⁸ group.

[5] The plant disease control agent according to [4], wherein in the formula (1),
X² is a hydrogen atom or a halogen atom,
A¹ is -CH₂-, -CH(CH₃)-, or methylene substituted with a C2-C5 alkoxycarbonyl group, and
R⁸ is a hydrogen atom or a C1-C4 alkyl group.

[6] A plant disease control method which comprises applying an effective amount of an amide compound represented by the formula (1) to plants or soil: 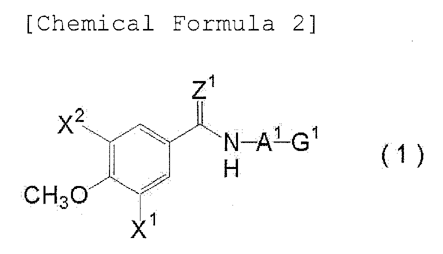 wherein
X¹ represents a fluorine atom or a methoxy group,
X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group or a CONR⁴R⁵ group, or a phenyl group optionally substituted with at least one member selected from the group consisting of a methyl group, a halogen atom, a cyano group and a nitro group,
Z¹ represents an oxygen atom or a sulfur atom,
A¹ represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G¹ represents a CR⁶R⁷R⁸ group (provided that the aforementioned A¹ is not a single bond when G¹ is a CR⁶R⁷R⁸ group), a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ represents a C1-C4 alkyl group, a C3-C4 alkenyl group or a C3-C4 alkynyl group,
R⁴ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a hydrogen atom, a C1-C4 alkyl group, a C3-C4 alkenyl group, a C3-C4 alkynyl group or a C2-C4 haloalkyl group,
R⁶ and R⁷ independently represent a C1-C4 alkyl group,
R⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR⁹R¹⁰ group, in which R⁹ and R¹⁰ are as defined above.

[7] The plant disease control method according to [6], wherein in the formula (1),
A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming ring is replaced by a carbonyl group, or a C3-C6 hydroxyiminocycloalkyl group optionally substituted with at least one member selected from the group [a-1].

[8] The plant disease control method according to [7], wherein in the formula (1),
X² is a hydrogen atom or a halogen atom, and
G¹ is a C3-C6 cycloalkyl group optionally substituted with at least one member selected from the group consisting of a halogen atom, a C1-C4 alkyl group and a hydroxyl group.

[9] The plant disease control method according to [6], wherein in the formula (1),
A¹ is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a CR⁶R⁷R⁸ group.

[10] The plant disease control method according to [9], wherein in the formula (1),
X² is a hydrogen atom or a halogen atom,
A¹ is -CH₂-, -CH(CH₃)-, or methylene substituted with a C2-C5 alkoxycarbonyl group, and
R⁸ is a hydrogen atom or a C1-C4 alkyl group.

[11] Use of an amide compound represented by the formula (1): 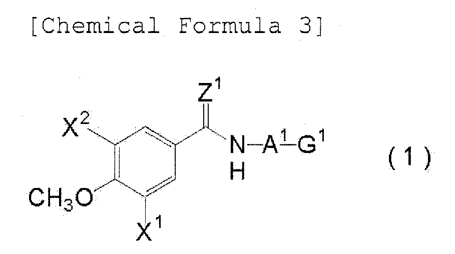 wherein
X¹ represents a fluorine atom or a methoxy group,
X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group or a CONR⁴R⁵ group, or a phenyl group optionally substituted with at least one member selected from the group consisting of a methyl group, a halogen atom, a cyano group and a nitro group,
Z¹ represents an oxygen atom or a sulfur atom,
A¹ represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G¹ represents a CR⁶R⁷R⁸ group (provided that the aforementioned A¹ is not a single bond when G¹ is a CR⁶R⁷R⁸ group), a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylenes forming ring is substituted with a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ represents a C1-C4 alkyl group, a C3-C4 alkenyl group or a C3-C4 alkynyl group,
R⁴ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a hydrogen atom, a C1-C4 alkyl group, a C3-C4 alkenyl group, a C3-C4 alkynyl group or a C2-C4 haloalkyl group,
R⁶ and R⁷ independently represent a C1-C4 alkyl group,
R⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR⁹R¹⁰ group, in which R⁹ and R¹⁰ are as defined above, for controlling plant diseases by applying it to plants or soil.

[12] The use of an amide compound according to [11], wherein in the formula (1),
A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming ring is replaced by a carbonyl group, or a C3-C6 hydroxyiminocycloalkyl group optionally substituted with at least one member selected from the group [a-1].

[13] The use of an amide compound according to [12], wherein in the formula (1),
X² is a hydrogen atom or a halogen atom, and
G¹ is a C3-C6 cycloalkyl group optionally substituted with at least one member selected from the group consisting of a halogen atom, a C1-C4 alkyl group and a hydroxyl group.

[14] The use of an amide compound according to [11], wherein in the formula (1),
A¹ is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a CR⁶R⁷R⁸ group.

[15] The use of an amide compound according to [14], wherein in the formula (1),
X² is a hydrogen atom or a halogen atom,
A¹ is -CH₂-, -CH(CH₃)-, or methylene substituted with a C2-C5 alkoxycarbonyl group, and
R⁸ is a hydrogen atom or a C1-C4 alkyl group.

[16] A 3,5-difluoro-4-methoxybenzamide compound represented by the formula (A): 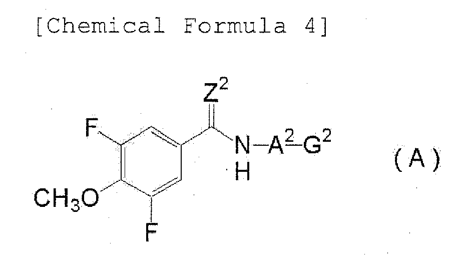 wherein
Z² represents an oxygen atom or a sulfur atom,
A² represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G² is a CR²⁶R²⁷R²⁸ group (provided that the aforementioned A² is not a single bond when G² is a CR²⁶R²⁷R²⁸ group), a C3-C6 cycloalkyl group optionally substituted with at least one member selected from the group [a-2] shown below, a C3-C6 cycloalkenyl group optionally substituted with at least one member selected from the group [a-2] shown below, a C3-C6 cycloalkyl group optionally substituted with at least one member selected from the group [a-2] shown below wherein one methylene forming ring is replaced by a carbonyl group, or a C3-C6 hydroxyiminocycloalkyl group optionally substituted with at least one member selected from the group [a-2] shown below,
R²⁶ and R²⁷ independently represent a C1-C4 alkyl group,
R²⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group, or an NR²⁹R³⁰ group, in which R²⁹ and R³⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-2]:
a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group, and an NR²⁹R³⁰ group, in which R²⁹ and R³⁰ are as defined above.

[17] The 3,5-difluoro-4-methoxybenzamide compound according to [16], wherein in the formula (A), G² is a C3-C6 cycloalkyl group optionally substituted with at least one member selected from the group [a-2], a C3-C6 cycloalkenyl group optionally substituted with at least one member selected from the group [a-2], a C3-C6 cycloalkyl group optionally substituted with at least one member selected from the group [a-2] wherein one methylenes forming ring is replaced by a carbonyl group, or a C3-C6 hydroxyiminocycloalkyl group optionally substituted with at least one member selected from the group [a-2].

[18] The 3,5-difluoro-4-methoxybenzamide compound according to [17], wherein G² is a C3-C6 cycloalkyl group optionally substituted with at least one member selected from the group consisting of a halogen atom, a C1-C4 alkyl group and a hydroxyl group.

[19] A 3,5-difluoro-4-methoxybenzamide compound wherein in the formula (A),
A² is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G² is a CR²⁶R²⁷R²⁸ group.

[20] The 3,5-difluoro-4-methoxybenzamide compound according to [19], wherein R²⁸ is a hydrogen atom or a C1-C4 alkyl group.

[21] The 3,5-difluoro-4-methoxybenzamide compound according to [19] or [20], wherein Z² is an oxygen atom and A² is -CH(CH₃)-.

### Effect of the Invention

According to the present invention, plant diseases can be controlled.

### Best Mode for Carrying Out the Invention

The plant disease control agent of the present invention contains an amide compound represented by the above formula (1).
In the formula (1), X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group, a CONR⁴R⁵ group, or a phenyl group optionally substituted with at least one member selected from the group consisting of a methyl group, a halogen atom, a cyano group and a nitro group.
Herein, the "optionally substituted with" means that one or more hydrogen atoms may be replaced by the listed substituents unless otherwise specified.

The halogen atom represented by X² in the formula (1) include a fluorine atom, a chlorine atom, a bromine atom and an iodine atom.
Examples of the C1-C4 alkyl group represented by X² include a methyl group, an ethyl group, a 1-methylethyl group, a 1,1-dimethylethyl group, a propyl group, and a 1-methylpropyl group.
Examples of the C2-C4 alkenyl group represented by X² include a vinyl group, a 1-propenyl group, a 2-propenyl group, a 2-butenyl group, and a 3-butenyl group.
Examples of the C2-C4 alkynyl group represented by X² include an ethynyl group, a 1-propynyl group, a 2-propynyl group, and a 3-butynyl group.
Examples of the C1-C4 haloalkyl group represented by X² include a fluoromethyl group, a chloromethyl group, a bromomethyl group, a difluoromethyl group, a dichloromethyl group, a dibromomethyl group, a trifluoromethyl group, a trichloromethyl group, a dichlorofluoromethyl group, a chlorodifluoromethyl group, a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C1-C4 alkoxy group represented by X² include a methoxy group, an ethoxy group, a 1-methylethoxy group, a 1,1-dimethylethoxy group, a propoxy group, a 1-methylpropoxy group, a 2-methylpropoxy group, and a butoxy group.
Examples of the C1-C4 alkylthio group represented by X² include a methylthio group, an ethylthio group, a 1-methylethylthio group, a 1,1-dimethylethylthio group, a propylthio group, and a 1-methylpropylthio group.
Examples of the C1-C4 hydroxyalkyl group represented by X² include a hydroxymethyl group, a 1-hydroxyethyl group, and a 2-hydroxyethyl group.
Examples of the phenyl group optionally substituted with at least one member selected from the group consisting of a methyl group, a halogen atom, a cyano group and a nitro group, which is represented by X², include a phenyl group, a 2-methylphenyl group, a 3-methylphenyl group, a 4-methylphenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group, and a 4-nitrophenyl group.

Examples of the C1-C4 alkyl group represented by R¹ or R² in the NR¹R² group include a methyl group, an ethyl group, a 1-methylethyl group, a 1,1-dimethylethyl group, a propyl group, and a 1-methylpropyl group.
Examples of the C3-C4 alkenyl group represented by each of the R¹ or R² include a 1-propenyl group, a 2-propenyl group, a 2-butenyl group, and a 3-butenyl group.
Examples of the C3-C4 alkynyl group represented by each of the R¹ or R² include a 1-propynyl group, a 2-propynyl group, and a 3-butynyl group.
Examples of the C2-C4 haloalkyl group represented by each of the R¹ or R² include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by each of the R¹ or R² include an acetyl group, an ethylcarbonyl group, a 1-methylethylcarbonyl group, and a 1,1-dimethylethylcarbonyl group.
Example of C2-C5 alkoxycarbonyl group represented by each of the R¹ or R² include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by each of the R¹ or R² include a methylsulfonyl group, an ethylsulfonyl group, a 1-methylethylsulfonyl group, and a 1,1-dimethylethylsulfonyl group.

Examples of the NR¹R² group include an amino group, a methylamino group, a dimethylamino group, an ethylamino group, a 2-propenylamino group, a 2-propynylamino group, a 2-chloroethylamino group, an acetylamino group, a propionylamino group, a 1,1-dimethylethylcarbonylamino group, a methoxycarbonylamino group, an ethoxycarbonylamino group, a methanesulfonylamino group, an N-acetyl-N-methylamino group, an N-ethoxycarbonyl-N-methylamino group, and a methanesulfonylmethylamino group.

Examples of the C1-C4 alkyl group represented by the R³ in CO₂R³ include a methyl group, an ethyl group, a 1-methylethyl group, a 1,1-dimethylethyl group, a propyl group, and a 1-methylpropyl group.
Examples of the C3-C4 alkenyl group represented by the R³ include a 1-propenyl group, a 2-propenyl group, a 2-butenyl group, and a 3-butenyl group.
Examples of the C3-C4 alkynyl group represented by the R³ include a 1-propynyl group, a 2-propynyl group, and a 3-butynyl group.

Examples of the C1-C4 alkyl group represented by the R⁴ in CONR⁴R⁵ include a methyl group, an ethyl group, a 1-methylethyl group, a 1,1-dimethylethyl group, a propyl group, and a 1-methylpropyl group.

Examples of the C3-C4 alkenyl group represented by the R⁴ include a 1-propenyl group, a 2-propenyl group, a 2-butenyl group, and a 3-butenyl group.
Examples of the C3-C4 alkynyl group represented by the R⁴ include a 1-propynyl group, a 2-propynyl group, and a 3-butynyl group.
Examples of the C2-C4 haloalkyl group represented by the R⁴ include a 1,1-difluoroethyl group, a 2,2,2-trifluoroethyl group, a 2-fluoroethyl group, a 3-fluoropropyl group, a 4-fluorobutyl group, and a 1-chloroethyl group.
Examples of the C2-C5 alkylcarbonyl group represented by the R⁴ include an acetyl group, an ethylcarbonyl group, a 1-methylethylcarbonyl group, and a 1,1-dimethylethylcarbonyl group.
Examples of the C2-C5 alkoxycarbonyl group represented by the R⁴ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by the R⁴ include a methylsulfonyl group, an ethylsulfonyl group, a 1-methylethylsulfonyl group, and a 1,1-dimethylethylsulfonyl group.

Examples of the C1-C4 alkyl group represented by the R⁵ include a methyl group, an ethyl group, a 1-methylethyl group, a 1,1-dimethylethyl group, a propyl group, and a 1-methylpropyl group.
Examples of the C3-C4 alkenyl group represented by the R⁵ include a 1-propenyl group, a 2-propenyl group, a 2-butenyl group, and a 3-butenyl group.
Examples of the C3-C4 alkynyl group represented by the R⁵ include a 1-propynyl group, a 2-propynyl group, and a 3-butynyl group.
Examples of the C2-C4 haloalkyl group represented by the R⁵ include a 1,1-difluoroethyl group, a 2,2,2-trifluoroethyl group, a 2-fluoroethyl group, a 3-fluoropropyl group, a 4-fluorobutyl group, and a 1-chloroethyl group.

Examples of the CONR⁴R⁵ group include a carbamoyl group, a methylcarbamoyl group, a dimethylcarbamoyl group, an ethylmethylcarbamoyl group, a (2-propenyl)carbamoyl group, a (2-propynyl)carbamoyl group, and a 2-chloroethylcarbamoyl group.

In the formula (1), A¹ represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group (hereinafter this methylene is referred to as a "substituted methylene").

Examples of the C1-C3 haloalkyl group for the substituted methylene represented by the aforementioned A¹ include a fluoromethyl group, a chloromethyl group, a bromomethyl group, a difluoromethyl group, a dichloromethyl group, a dibromomethyl group, a trifluoromethyl group, a trichloromethyl group, a dichlorofluoromethyl group, a chlorodifluoromethyl group, a 1,1-difluoroethyl group, a 2,2,2-trifluoroethyl group, a 2-fluoroethyl group, a 3-fluoropropyl group, and a 1-chloroethyl group.
Examples of the C2-C4 alkenyl group for the substituted methylene include a vinyl group, a 1-propenyl group, a 2-propenyl group, a 2-butenyl group, and a 3-butenyl group.
Examples of the C2-C4 alkynyl group for the substituted methylene include an ethynyl group, a 1-propynyl group, a 2-propynyl group, a 3-butynyl group.
Examples of the C2-C5 alkoxycarbonyl group in the substituted methylene include a methoxycarbonyl group, an ethoxycarbonyl group, a 1-methylethoxycarbonyl group, and a 1,1-dimethylethoxycarbonyl group.

Specific examples of the substituted methylene include 2,2,2-trifluoroethane-1,1-diyl, 2,2-difluoroethane-1,1-diyl, 2-fluoroethane-1,1-diyl, 3,3,3-trifluoropropane-1,1-diyl, 2,2,2-trichloroethane-1,1-diyl, 2,2-dichloroethanediyl, 2-chloroethane-1,1-diyl, 2-propene-1,1-diyl, 2-butene-1,1-diyl, 3-methyl-2-butene-1,1-diyl, 2-propyne-1,1-diyl, 2-butyne-1,1-diyl, 3-butyne-1,1-diyl, cyanomethylene, (methoxycarbonyl)methylene, (ethoxycarbonyl)methylene, and (1-methylethoxycarbonyl)methylene.

In the present invention, G¹ represents a CR⁶R⁷R⁸ group (provided that A¹ is not a single bond when G¹ is a CR⁶R⁷R⁸ group), a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming ring is replaced by a carbonyl group (hereinafter, this cycloalkyl group is referred to as a "substituted cycloalkyl group"), or a C3-C6 hydroxyiminocycloalkyl group optionally substituted with at least one member selected from the group [a-1].
Herein, the "optionally substituted with at least one member selected from the group [a-1]" may be abbreviated to "may be substituted with a member of the group [a-1]".

In the CR⁶R⁷R⁸ group, R⁶ and R⁷ independently represent a C1-C4 alkyl group.
Examples of the C1-C4 alkyl group represented by R⁶ or R⁷ include a methyl group, an ethyl group, a 1-methylethyl group, a 1,1-dimethylethyl group, a propyl group, and a 1-methylpropyl group.

In the CR⁶R⁷R⁸ group, R⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group, or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group.

Examples of the C1-C4 alkyl group represented by the R⁸ include a methyl group, an ethyl group, a 1-methylethyl group, a 1,1-dimethylethyl group, a propyl group, and a 1-methylpropyl group.
Examples of the C2-C4 alkenyl group represented by the R⁸ include a vinyl group, a 1-propenyl group, a 2-propenyl group, a 2-butenyl group, and a 3-butenyl group.
Examples of the C2-C4 alkynyl group represented by the R⁸ include an ethynyl group, a 1-propynyl group, a 2-propynyl group, and a 3-butynyl group.
Examples of the C2-C5 alkoxycarbonyl group represented by the R⁸ include a methoxycarbonyl group, an ethoxycarbonyl group, a 1-methylethoxycarbonyl group, and a 1,1-dimethylethoxycarbonyl group.

The halogen atom represented by the R⁸ include a fluorine atom, a chlorine atom, a bromine atom, and an iodine atom.
Examples of the C1-C6 alkoxy group represented by the R⁸ include a methoxy group, an ethoxy group, a propoxy group, an isopropoxy group, a butoxy group, an isobutoxy group, a sec-butoxy group, a tert-butoxy group, a pentyloxy group, an isopentyloxy group, and a hexyloxy group.
Examples of the C3-C6 alkenyloxy group represented by the R⁸ include a 2-propenyloxy group, a 1-methyl-2-propenyloxy group, a 2-methyl-2-propenyloxy group, a 2-butenyloxy group, a 3-butenyloxy group, a 2-hexenyloxy group, and a 5-hexenyloxy group.
Examples of the C1-C6 haloalkyl group represented by the R⁸ include a fluoromethyl group, a difluoromethyl group, a trifluoromethyl group, a trichloromethyl group, a chlorofluoromethyl group, a bromodifluoromethyl group, a 2-fluoroethyl group, a 2,2-difluoroethyl group, a 2,2,2-trifluoroethyl group, and a 6,6,6-trifluorohexyl group.

Examples of the C2-C6 haloalkoxy group represented by the R⁸ include a 2-fluoroethoxy group, a 2-chloroethoxy group, a 2,2,2-trifluoroethoxy group, a 1,1,2,2-tetrafluoroethoxy group, a 5-chloropentyloxy group, a 4-fluoroisopentyloxy group, and a 2,2-dichlorohexyloxy group.
Examples of the C1-C3 alkylthio group represented by the R⁸ include a methylthio group, an ethylthio group, a 1-methylethylthio group, and a propylthio group.
Examples of the C1-C6 hydroxyalkyl group represented by the R⁸ include a hydroxymethyl group, a 1-hydroxyethyl group, a 2-hydroxyethyl group, a 1-hydroxypropyl group, and a 2-hydroxypropyl group.
Examples of the C2-C4 alkylcarbonyloxy group represented by the R⁸ include an acetoxy group, an ethylcarbonyloxy group, a 1-methylethylcarbonyloxy group, and a propylcarbonyloxy group.

Examples of the (C1-C3 alkylamino)C1-C6 alkyl group represented by the R⁸ include an N-methylaminomethyl group, an N-ethylaminomethyl group, a 1-(N-methylamino)ethyl group, a 2-(N-methylamino)ethyl group, and a 1-(N-ethylamino)ethyl group.
Examples of the (di(C1-C3 alkyl)amino)C1-C6 alkyl group represented by the R⁸ include an N,N-dimethylaminomethyl group, a 1-(N,N-dimethylamino)ethyl group, a 2-(N,N-dimethylamino)ethyl group, and an N,N-diethylaminomethyl group.
Examples of the C2-C6 cyanoalkyl group represented by the R⁸ include a cyanomethyl group, a 1-cyanoethyl group, and a 2-cyanoethyl group.
Examples of the C1-C3 alkylsulfonyl group represented by the R⁸ include a methanesulfonyl group, and an ethanesulfonyl group.

The group [a-1] for G¹ consists of the following substituents:
a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)Cl-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group, and an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group.

The halogen atom in the group [a-1] include a fluorine atom, a chlorine atom, a bromine atom and an iodine atom.
Examples of the C1-C4 alkyl group in the group [a-1] include a methyl group, an ethyl group, a 1-methylethyl group, a 1,1-dimethylethyl group, a propyl group, and a 1-methylpropyl group.
Examples of the C2-C4 alkenyl group in the group [a-1] include a vinyl group, a 1-propenyl group, a 2-propenyl group, a 2-butenyl group, and a 3-butenyl group.
Examples of the C2-C4 alkynyl group in the group [a-1] include an ethynyl group, a 1-propynyl group, a 2-propynyl group, and a 3-butynyl group.
Examples of the C2-C5 alkoxycarbonyl group in the group [a-1] include a methoxycarbonyl group, an ethoxycarbonyl group, a 1-methylethoxycarbonyl group, and a 1,1-dimethylethoxycarbonyl group.
Examples of the C1-C6 alkoxy group in the group [a-1] include a methoxy group, an ethoxy group, a propoxy group, an isopropoxy group, a butoxy group, an isobutoxy group, a sec-butoxy group, a tert-butoxy group, a pentyloxy group, an isopentyloxy group, and a hexyloxy group.
Examples of the C3-C6 alkenyloxy group in the group [a-1] include a 2-propenyloxy group, a 1-methyl-2-propenyloxy group, a 2-methyl-2-propenyloxy group, a 2-butenyloxy group, a 3-butenyloxy group, a 2-hexenyloxy group, and a 5-hexenyloxy group.

Examples of the C1-C6 haloalkyl group in the group [a-1] include a fluoromethyl group, a difluoromethyl group, trifluoromethyl group, a trichloromethyl group, a chlorofluoromethyl group, a bromodifluoromethyl group, a 2-fluoroethyl group, a 2,2-difluoroethyl group, a 2,2,2-trifluoroethyl group, and a 6,6,6-trifluorohexyl group.
Examples of the C1-C6 haloalkoxy group in the group [a-1] include a trifluoromethoxy group, a difluoromethoxy group, a bromodifluoromethoxy group, a chlorodifluoromethoxy group, a fluoromethoxy group, a 2,2,2-trifluoroethoxy group, a 1,1,2,2-tetrafluoroethoxy group, a 5-chloropentyloxy group, a 4-fluoroisopentyloxy group, and a 2,2-dichlorohexyloxy group.
Examples of the C1-C3 alkylthio group in the group [a-1] include a methylthio group, an ethylthio group, a 1-methylethylthio group, and a propylthio group.
Examples of the C1-C3 alkylidene group which forms a double bond with a ring-forming carbon atom in the group [a-1] include a methylene group which forms a double bond with a ring-forming carbon atom, an ethylidene group which forms a double bond with a ring-forming carbon atom, an isopropylidene group which forms a double bond with a ring-forming carbon atom, and a propylidene group which forms a double bond with a ring-forming carbon atom.

Examples of the C1-C6 hydroxyalkyl group in the group [a-1] include a hydroxymethyl group, a 1-hydroxyethyl group, a 2-hydroxyethyl group, a 1-hydroxypropyl group, and a 2-hydroxypropyl group.
Examples of the C2-C4 alkylcarbonyloxy group in the group [a-1] include an acetoxy group, an ethylcarbonyloxy group, a 1-methylethylcarbonyloxy group, and a propylcarbonyloxy group.
Examples of the (C1-C3 alkylamino)C1-C6 alkyl group in the group [a-1] include an N-methylaminomethyl group, an N-ethylaminomethyl group, a 1-(N-methylamino)ethyl group, a 2-(N-methylamino)ethyl group, and a 1-(N-ethylamino)ethyl group.
Examples of the (di(C1-C3 alkyl)amino)C1-C6 alkyl group in the group [a-1] include an N,N-dimethylaminomethyl group, a 1-(N,N-dimethylamino)ethyl group, a 2-(N,N-dimethylamino)ethyl group, and an N,N-diethylaminomethyl group.
Examples of the C2-C6 cyanoalkyl group in the group [a-1] include a cyanomethyl group, a 1-cyanoethyl group, and a 2-cyanoethyl group.
Examples of the C1-C3 alkylsulfonyl group in the group [a-1] include a methanesulfonyl group, and an ethanesulfonyl group.

Examples of the C3-C6 cycloalkyl group which may be substituted with a member of the group [a-1] include a cyclopropyl group, a cyclobutyl group, a cyclopentyl group and a cyclohexyl group.
Examples of the C3-C6 cycloalkenyl group which may be substituted with a member of the group [a-1] include a 2-cyclopropenyl group, a 1-cyclobutenyl group, a 2-cyclobutenyl group, a 1-cyclopentenyl group, a 2-cyclopentenyl group, a 3-cyclopentenyl group, a 1-cyclohexenyl group, a 2-cyclohexenyl group and a 3-cyclohexenyl group.
Examples of the substituted cycloalkyl group include a 2-oxocyclopropyl group, a 2-oxocyclobutyl group, a 3-oxocyclobutyl group, a 2-oxocyclopentyl group, a 3-oxocyclopentyl group, a 2-oxocyclohexyl group, a 3-oxocyclohexyl group and a 4-oxocyclohexyl group.
Examples of the C3-C6 hydroxyiminocycloalkyl group which may be substituted with a member of the group [a-1] include a 2-hydroxyiminocyclopropyl group, a 2-hydroxyiminocyclobutyl group, a 3-hydroxyiminocyclobutyl group, a 2-hydroxyiminocyclopentyl group, a 3-hydroxyiminocyclopentyl group, a 2-hydroxyiminocyclohexyl group, a 3-hydroxyiminocyclohexyl group, and a 4-hydroxyiminocyclohexyl group.

Examples of C1-C4 alkyl group represented by the R⁹ in NR⁹R¹⁰ include a methyl group, an ethyl group, a 1-methylethyl group, a 1,1-dimethylethyl group, a propyl group, and a 1-methylpropyl group.
Examples of the C2-C5 alkylcarbonyl group represented by the R⁹ include an acetyl group, an ethylcarbonyl group, a 1-methylethylcarbonyl group, and a 1,1-dimethylethylcarbonyl group.
Examples of the C2-C5 alkoxycarbonyl group represented by the R⁹ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by the R⁹ include a methylsulfonyl group, an ethylsulfonyl group, a 1-methylethylsulfonyl group, and a 1,1-dimethylethylsulfonyl group.

Examples of the C1-C4 alkyl group represented by R¹⁰ in NR⁹R¹⁰ include a methyl group, an ethyl group, a 1-methylethyl group, a 1,1-dimethylethyl group, a propyl group, and a 1-methylpropyl group.
Examples of the C2-C5 alkylcarbonyl group represented by the R¹⁰ include an acetyl group, an ethylcarbonyl group, a 1-methylethylcarbonyl group, and a 1,1-dimethylethylcarbonyl group.
Examples of the C2-C5 alkoxycarbonyl group represented by the R¹⁰ include a methoxycarbonyl group, an ethoxycarbonyl group, a 1-methylethoxycarbonyl group, and a 1,1-dimethylethoxycarbonyl group.
Examples of the C1-C4 alkylsulfonyl group represented by the R¹⁰ include a methylsulfonyl group, an ethylsulfonyl group, a 1-methylethylsulfonyl group, and a 1,1-dimethylethylsulfonyl group.

Examples of NR⁹R¹⁰ in the group [a-1] include an amino group, a methylamino group, a dimethylamino group, an ethylamino group, an acetylamino group, a propionylamino group, a 1,1-dimethylethylcarbonylamino group, a methoxycarbonylamino group, an ethoxycarbonylamino group, a 1,1-dimethylethoxycarbonylamino group, a methanesulfonylamino group, an N-acetyl-N-methylamino group, an N-ethoxycarbonyl-N-methylamino group, and a methanesulfonylmethylamino group.

Examples of a group represented by A¹-G¹ include: a 2-methylpropyl group, a 1,2-dimethylpropyl group, a 2,2-dimethylpropyl group, a 1,2,2-trimethylpropyl group, a 1,1,2,2-tetramethylpropyl group, a 2-methyl-2-hydroxypropyl group, a 1,2-dimethyl-2-hydroxypropyl group, a 2-chloro-1,2-dimethylpropyl group, a 2-chloro-2-methylpropyl group, a 1,2-dimethylbutyl group, a 2,2-dimethyl-3-hydroxypropyl group, a 3-hydroxy-1,2,2-trimethylpropyl group;

a cyclopropyl group, a 2,2,3,3-tetramethylcyclopropyl group;
a cyclobutyl group;
a cyclopentyl group, a 2-methylcyclopentyl group, a 2-hydroxycyclopentyl group, a 2-chlorocyclopentyl group, a 2-bromocyclopentyl group, a 2,2-dimethylcyclopentyl group, a 2-fluoro-2-methylcyclopentyl group, a 2-chloro-2-methylcyclopentyl group, a 2-hydroxy-2-methylcyclopentyl group, a 2,2-difluorocyclopentyl group, a 3-methylcyclopentyl group;
a cyclohexyl group, a 1-methylcyclohexyl group, a 2-methylcyclohexyl group, a 3-methylcyclohexyl group, a 4-methylcyclohexyl group, a 2-trifluoromethylcyclohexyl group, a 2,2-dimethylcyclohexyl group, a 2-fluoro-2-methylcyclohexyl group, a 2-chloro-2-methylcyclohexyl group, a 2-hydroxy-2-methylcyclohexyl group, a 2,2-difluorocyclohexyl group, a 2,3-dimethylcyclohexyl group, a 2,6-dimethylcyclohexyl group, a 2-ethylcyclohexyl group, a 2-chlorocyclohexyl group, a 2-fluorocyclohexyl group, a 2-bromocyclohexyl group, a 2-iodocyclohexyl group, a 2-hydroxycyclohexyl group, a 1-cyanocyclohexyl group, a 2-cyanocyclohexyl group, a 1-carboxycyclohexyl group, a 1-(methoxycarbonyl)cyclohexyl group, a 1-(ethoxycarbonyl)cyclohexyl group, a 2-(methoxycarbonyl)cyclohexyl group, a 2-(ethoxycarbonyl)cyclohexyl group;

a cyclopropylmethyl group, a (1-methylcyclopropyl)methyl group, a (2-methylcyclopropyl)methyl group, a (1-hydroxycyclopropyl)methyl group, a (2-hydroxycyclopropyl)methyl group, a (2,2,3,3-tetramethylcyclopropyl)methyl group, a (1-fluorocyclopropyl)methyl group, a (1-chlorocyclopropyl)methyl group, a (1-cyanocyclopropyl)methyl group, a (1-ethoxycarbonylcyclopropyl)methyl group, a (1-methoxycarbonylcyclopropyl)methyl group; a cyclobutylmethyl group, a (1-methylcyclobutyl)methyl group, a (2-methylcyclobutyl)methyl group, a (3-methylcyclobutyl)methyl group, a (1-hydroxycyclobutyl)methyl group, a (2-hydroxycyclobutyl)methyl group, a (3-hydroxycyclobutyl)methyl group, a (1-fluorocyclobutyl)methyl group, a (1-chlorocyclobutyl)methyl group, (1-cyanocyclobutyl)methyl group, a (1-ethoxycarbonylcyclobutyl)methyl group, a (1-methoxycarbonylcyclobutyl)methyl group; a cyclopentylmethyl group, a (1-methylcyclopentyl)methyl group, a (2-methylcyclopentyl)methyl group, a (3-methylcyclopentyl)methyl group, a (1-hydroxycyclopentyl)methyl group, a (2-hydroxycyclopentyl)methyl group, a (3-hydroxycyclopentyl)methyl group, a (1-fluorocyclopentyl)methyl group, a (1-chlorocyclopentyl)methyl group, a (1-cyanocyclopentyl)methyl group, a (1-ethoxycarbonylcyclopentyl)methyl group, a (1-methoxycarbonylcyclopentyl)methyl group;

a cyclohexylmethyl group, a (1-methylcyclohexyl)methyl group, a (2-methylcyclohexyl)methyl group, a (3-methylcyclohexyl)methyl group, a (4-methylcyclohexyl)methyl group, a (2,3-dimethylcyclohexyl)methyl group, a (1-hydroxycyclohexyl)methyl group, a (2-hydroxycyclohexyl)methyl group, a (3-hydroxycyclohexyl)methyl group, a (4-hydroxycyclohexyl)methyl group, a (1-fluorocyclohexyl)methyl group, a (1-chlorocyclohexyl)methyl group, (1-cyanocyclohexyl)methyl group, a (1-ethoxycarbonylcyclohexyl)methyl group, a (1-methoxycarbonylcyclohexyl)methyl group, a (1-ethynylcyclohexyl)methyl group, a (2-fluorocyclohexyl)methyl group, a (2-chlorocyclohexyl)methyl group, (2-cyanocyclohexyl)methyl group, a (2-ethoxycarbonylcyclohexyl)methyl group, a (2-methoxycarbonylcyclohexyl)methyl group;

a 1-(cyclopropyl)ethyl group, a 1-(1-methylcyclopropyl)ethyl group, a 1-(2-methylcyclopropyl)ethyl group, a 1-(1-hydroxycyclopropyl)ethyl group, a 1-(2-hydroxycyclopropyl)ethyl group, a 1-(2,2,3,3-tetramethylcyclopropyl)ethyl group, a 1-(1-fluorocyclopropyl)ethyl group, a 1-(1-chlorocyclopropyl)ethyl group, a 1-(1-cyanocyclopropyl)ethyl group, a 1-(1-ethoxycarbonylcyclopropyl)ethyl group, a 1-(1-methoxycarbonylcyclopropyl)ethyl group;
a 1-(cyclobutyl)ethyl group, a 1-(1-methylcyclobutyl)ethyl group, a 1-(2-methylcyclobutyl)ethyl group, a 1-(3-methylcyclobutyl)ethyl group, a 1-(1-hydroxycyclobutyl)ethyl group, a 1-(2-hydroxycyclobutyl)ethyl group, a 1-(3-hydroxycyclobutyl)ethyl group, a 1-(1-fluorocyclobutyl)ethyl group, a 1-(1-chlorocyclobutyl)ethyl group, a 1-(1-cyanocyclobutyl)ethyl group, a 1-(1-ethoxycarbonylcyclobutyl)ethyl group, a 1-(1-methoxycarbonylcyclobutyl)ethyl group;

a 1-(cyclopentyl)ethyl group, a 1-(1-methylcyclopentyl)ethyl group, a 1-(2-methylcyclopentyl)ethyl group, a 1-(3-methylcyclopentyl)ethyl group, a 1-(1-hydroxycyclopentyl)ethyl group, a 1-(2-hydroxycyclopentyl)ethyl group, a 1-(3-hydroxycyclopentyl)ethyl group, a 1-(1-fluorocyclopentyl)ethyl group, a 1-(1-chlorocyclopentyl)ethyl group, a 1-(1-cyanocyclopentyl)ethyl group, a 1-(1-ethoxycarbonylcyclopentyl)ethyl group, a 1-(1-methoxycarbonylcyclopentyl)ethyl group;
a 1-(cyclohexyl)ethyl group, a 1-(1-methylcyclohexyl)ethyl group, a 1-(2-methylcyclohexyl)ethyl group, a 1-(3-methylcyclohexyl)ethyl group, a 1-(4-methylcyclohexyl)ethyl group, a 1-(2,3-dimethylcyclohexyl)ethyl group, a 1-(1-hydroxycyclohexyl)ethyl group, a 1-(2-hydroxycyclohexyl)ethyl group, a 1-(3-hydroxycyclohexyl)ethyl group, a 1-(4-hydroxycyclohexyl)ethyl group, a 1-(1-fluorocyclohexyl)ethyl group, a 1-(1-chlorocyclohexyl)ethyl group, 1-(1-cyanocyclohexyl)ethyl group, a 1-(1-ethoxycarbonylcyclohexyl)ethyl group, a 1-(1-methoxycarbonylcyclohexyl)ethyl group, a 1-(1-ethynylcyclohexyl)ethyl group, a 1-(2-fluorocyclohexyl)ethyl group, a 1-(2-chlorocyclohexyl)ethyl group, a 1-(2-cyanocyclohexyl)ethyl group, a 1-(2-ethoxycarbonylcyclohexyl)ethyl group, a 1-(2-methoxycarbonylcyclohexyl)ethyl group;

a 1-methyl-1-cyclopropylethyl group, a 1-methyl-1-cyclobutylethyl group, a 1-methyl-1-(1-hydroxycyclobutyl)ethyl group, a 1-methyl-1-(1-fluorocyclobutyl)ethyl group, a 1-methyl-1-cyclopentylethyl group, a 1-methyl-1-(1-hydroxycyclopentyl)ethyl group, a 1-methyl-1-(1-fluorocyclopentyl)ethyl group, a 1-methyl-1-cyclohexylethyl group, a 1-methyl-1-(1-hydroxycyclohexyl)ethyl group and a 1-methyl-1-(1-fluorocyclohexyl)ethyl group, a (1-hydroxymethylcyclopropyl)methyl group, a (1-hydroxymethylcyclobutyl)methyl group, a (1-hydroxymethylcyclopentyl)methyl group, a (1-hydroxymethylcyclohexyl)methyl group, a (1-cyclohexenyl)methyl group, a (2-cyclohexenyl)methyl group, a (3-cyclohexenyl)methyl group, a (1-cyclopentenyl)methyl group, a (2-cyclopentenyl)methyl group, a (1-dimethylaminocyclopropyl)methyl group, a (1-dimethylaminocyclobutyl)methyl group, a (1-dimethylaminocyclopentyl)methyl group, a (1-dimethylaminocyclohexyl)methyl group, a (1-acetoxycyclopropyl)methyl group, a (1-acetoxycyclobutyl)methyl group, a (1-acetoxycyclopentyl)methyl group, a (1-acetoxycyclohexyl)methyl group, a (2-acetoxycyclohexyl)methyl group, a 2-cyclohexenyl group, a 3-cyclohexenyl group, a 2-methoxycyclopropyl group, a 2-methoxycyclobutyl group, a 2-methoxycyclopentyl group, a 2-methoxycyclohexyl group, a 3-methoxycyclohexyl group, a 4-methoxycyclohexyl group, a (1-methoxycyclohexyl)methyl group, a (2-methoxycyclohexyl)methyl group, a 2-acetoxycyclobutyl group, a 2-acetoxycyclopentyl group, a 2-acetoxycyclohexyl group, a 2-methylthiocyclobutyl group, a 2-methylthiocyclopentyl group, a 2-methylthiocyclohexyl group, a 2-(1,1-dimethylethoxycarbonylamino)cyclobutyl group, a 2-(1,1-dimethylethoxycarbonylamino)cyclopentyl group, a 2-(1,1-dimethylethoxycarbonylamino)cyclohexyl group, a 2-aminocyclobutyl group, a 2-aminocyclopentyl group, a 2-aminocyclohexyl group, a 2-acetylaminocyclobutyl group, a 2-acetylaminocyclopentyl group, a 2-acetylaminocyclohexyl group, a 2-dimethylaminocyclobutyl group, a 2-dimethylaminocyclopentyl group, a 2-dimethylaminocyclohexyl group, a 2-phenylcyclobutyl group, a 2-phenylcyclopentyl group, a 2-phenylcyclohexyl group, a 2-benzylcyclobutyl group, a 2-benzylcyclopentyl group, a 2-benzylcyclohexyl group, a 2-trifluoromethylcyclobutyl group, a 2-trifluoromethylcyclopentyl group, a 2-trifluoromethylcyclohexyl group, a 2-hydroxymethylcyclobutyl group, a 2-hydroxymethylcyclopentyl group, a 2-hydroxymethylcyclohexyl group, a 2-methylenecyclohexyl group, a 3-methylenecyclohexyl group, a 4-methylenecyclohexyl group, a 2-methylenecyclopentyl group, a 3-methylenecyclopentyl group, a 2-oxocyclohexyl group, 3-oxocyclohexyl group, a 4-oxocyclohexyl group, a 2-oxocyclopentyl group, a 3-oxocyclopentyl group, a 2-hydroxyiminocyclohexyl group, and a 2-hydroxyiminocyclopentyl group.

Examples of the amide compound represented by the formula (1) include the following aspects:
an amide compound of the formula (1), wherein X¹ is a fluorine atom;
an amide compound of the formula (1), wherein X¹ is a methoxy group;
an amide compound of the formula (1), wherein X² is a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group or a C1-C4 hydroxyalkyl group;
an amide compound of the formula (1), wherein X² is a hydrogen atom, a fluorine atom or a methoxy group;
an amide compound of the formula (1), wherein X² is a fluorine atom;
an amide compound of the formula (1), wherein X¹ is a fluorine atom, and X² is a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group or a C1-C4 hydroxyalkyl group;
an amide compound of the formula (1), wherein X¹ is a fluorine atom, and X² is a hydrogen atom, a fluorine atom or a methoxy group;
an amide compound of the formula (1), wherein X¹ is a methoxy group, and X² is a hydrogen atom, a fluorine atom or a methoxy group;
an amide compound of the formula (1), wherein X¹ is a fluorine atom, and X² is a hydrogen atom;
an amide compound of the formula (1), wherein X¹ is a fluorine atom, and X² is a fluorine atom;
an amide compound of the formula (1), wherein X¹ is a fluorine atom, and X² is a methoxy group;
an amide compound of the formula (1), wherein X¹ is a methoxy group, and X² is a hydrogen atom;
an amide compound of the formula (1), wherein X¹ is a methoxy group, and X² is a fluorine atom;
an amide compound of the formula (1), wherein X¹ is a methoxy group, and X² is a methoxy group;

an amide compound of the formula (1), wherein Z¹ is an oxygen atom;
an amide compound of the formula (1), wherein X¹ is a fluorine atom, and Z¹ is an oxygen atom;
an amide compound of the formula (1), wherein X¹ is a methoxy group, and Z¹ is an oxygen atom;
an amide compound of the formula (1), wherein X² is a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group or a C1-C4 hydroxyalkyl group, and Z¹ is an oxygen atom;
an amide compound of the formula (1), wherein X² is a hydrogen atom, a fluorine atom or a methoxy group, and Z¹ is an oxygen atom;
an amide compound of the formula (1), wherein X² is a fluorine atom, and Z¹ is an oxygen atom;
an amide compound of the formula (1), wherein X¹ is a fluorine atom, X² is a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group or a C1-C4 hydroxyalkyl group, and Z¹ is an oxygen atom;
an amide compound of the formula (1), wherein X¹ is a fluorine atom, X² is a hydrogen atom, a fluorine atom or a methoxy group, and Z¹ is an oxygen atom;
an amide compound of the formula (1), wherein X¹ is a methoxy group, X² is a hydrogen atom, a fluorine atom or a methoxy group, and Z¹ is an oxygen atom;
an amide compound of the formula (1), wherein X¹ is a fluorine atom, X² is a hydrogen atom, and Z¹ is an oxygen atom;
an amide compound of the formula (1), wherein X¹ is a fluorine atom, X² is a fluorine atom, and Z¹ is an oxygen atom;
an amide compound of the formula (1), wherein X¹ is a fluorine atom, X² is a methoxy group, and Z¹ is an oxygen atom;
an amide compound of the formula (1), wherein X¹ is a methoxy group, X² is a hydrogen atom, and Z¹ is an oxygen atom;
an amide compound of the formula (1), wherein X¹ is a methoxy group, X² is a fluorine atom, and Z¹ is an oxygen atom;
an amide compound of the formula (1), wherein X¹ is a methoxy group, X² is a methoxy group, and Z¹ is an oxygen atom;

an amide compound of the formula (1), wherein A¹ is a single bond, -CH₂- or -CH(CH₃)-;
an amide compound of the formula (1), wherein A¹ is a single bond;
an amide compound of the formula (1), wherein A¹ is -CH₂-; an amide compound of the formula (1), wherein A¹ is -CH(CH₃)-;
an amide compound of the formula (1), wherein G¹ is a C3-C6 cycloalkyl group which may be substituted with a member of the group [a-1];
an amide compound of the formula (1), wherein G¹ is a C3-C6 cycloalkyl group optionally substituted with at least one member selected from the group consisting of a halogen atom, a C1-C4 alkyl group, a C2-C4 alkenyl group, a C2-C4 alkynyl group, a hydroxyl group, a cyano group, a C1-C6 alkoxy group, a C1-C3 alkylidene group which forms a double bond with a ring-forming carbon atom, and a C1-C6 hydroxyalkyl group;
an amide compound of the formula (1), wherein G¹ is a C3-C6 cycloalkyl group optionally substituted with at least one member selected from the group consisting of a halogen atom, a C1-C4 alkyl group, a hydroxyl group and a cyano group;
an amide compound of the formula (1), wherein A¹-G¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group,
a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;
an amide compound of the formula (1), wherein A¹-G¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group;
an amide compound of the formula (1), wherein A¹-G¹ is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group, a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;

an amide compound of the formula (1), wherein A¹-G¹ is a 2-methylcyclohexyl group;
an amide compound of the formula (1), wherein A¹-G¹ is a 2-chlorocyclohexyl group;
an amide compound of the formula (1), wherein A¹-G¹ is a cyclohexylmethyl group;
an amide compound of the formula (1), wherein A¹-G¹ is a 1-cyclohexylethyl group;
an amide compound of the formula (1), wherein A¹-G¹ is a (1-hydroxycyclohexyl)methyl group;
an amide compound of the formula (1), wherein A¹-G¹ is a 1-(1-hydroxycyclohexyl)ethyl group;
an amide compound of the formula (1), wherein A¹-G¹ is a cyclobutylmethyl group;
an amide compound of the formula (1), wherein A¹-G¹ is a 1-cyclobutylethyl group;
an amide compound of the formula (1), wherein A¹-G¹ is a (1-hydroxycyclobutyl)methyl group;
an amide compound of the formula (1), wherein A¹-G¹ is a 1-(1-hydroxycyclobutyl)ethyl group;

an amide compound of the formula (1), wherein X¹ is a fluorine atom, Z¹ is an oxygen atom, and A¹ is a single bond;
an amide compound of the formula (1), wherein X¹ is a fluorine atom, Z¹ is an oxygen atom, and A¹ is -CH₂-;
an amide compound of the formula (1), wherein X¹ is a fluorine atom, Z¹ is an oxygen atom, and A¹ is -CH(CH₃)-; an amide compound of the formula (1), wherein X¹ is a fluorine atom, Z¹ is an oxygen atom, and G¹ is a C3-C6 cycloalkyl group optionally substituted with at least one member selected from the group [a-1];
an amide compound of the formula (1), wherein X¹ is a fluorine atom, Z¹ is an oxygen atom, and G¹ is a C3-C6 cycloalkyl group optionally substituted with at least one member selected from the group consisting of a halogen atom, a C1-C4 alkyl group, a C2-C4 alkenyl group, a C2-C4 alkynyl group, a hydroxyl group, a cyano group, a C1-C6 alkoxy group, a C1-C3 alkylidene group which forms a double bond with a ring-forming carbon atom, and a C1-C6 hydroxyalkyl group;
an amide compound of the formula (1), wherein X¹ is a fluorine atom, Z¹ is an oxygen atom, and G¹ is a C3-C6 fluorine atom, Z¹ is an oxygen atom, and G¹ is a C3-C6 cycloalkyl group optionally substituted with at least one member selected from the group consisting of a halogen atom, a C1-C4 alkyl group, a hydroxyl group and a cyano group;
an amide compound of the formula (1), wherein X¹ is a fluorine atom, Z¹ is an oxygen atom, and A¹-G¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group, a 2-hydroxycyclohexyl group, cyclobutylmethyl group, a cyclopentylmethyl group, cyclohexylmethyl group, a (1-hydroxycyclobutyl)methyl group, (1-hydroxycyclopentyl)methyl group, a (1-hydroxycyclohexyl)methyl group, a 1-cyclobutylethyl group,
a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;
an amide compound of the formula (1), wherein X¹ is a fluorine atom, Z¹ is an oxygen atom, and A¹-G¹ is a 2-methylcyclopentyl group, a 2-fluorocyclopentyl group, a 2-chlorocyclopentyl group, a 2-hydroxycyclopentyl group, a 2-methylcyclohexyl group, a 2-fluorocyclohexyl group, a 2-chlorocyclohexyl group or a 2-hydroxycyclohexyl group;
an amide compound of the formula (1), wherein A¹-G¹ is a cyclobutylmethyl group, a cyclopentylmethyl group, a cyclohexylmethyl group, a (1-hydroxycyclobutyl)methyl group, a (1-hydroxycyclopentyl)methyl group, a (1-hydroxycyclohexyl)methyl group, a 1-cyclobutylethyl group,
a 1-cyclopentylethyl group, a 1-cyclohexylethyl group, a 1-(1-hydroxycyclobutyl)ethyl group, a 1-(1-hydroxycyclopentyl)ethyl group or a 1-(1-hydroxycyclohexyl)ethyl group;

an amide compound of the formula (1), wherein X² is a hydrogen atom or a halogen atom, and G¹ is a C3-C6 cycloalkyl group optionally substituted with at least one member selected from the group consisting of a halogen atom, a C1-C4 alkyl group and a hydroxyl group;
an amide compound of the formula (1), wherein X¹ is a fluorine atom, X² is a fluorine atom, and G¹ is a C3-C6 cycloalkyl group optionally substituted with at least one member selected from the group consisting of a halogen atom, a C1-C4 alkyl group and a hydroxyl group;
an amide compound of the formula (1), wherein X² is a hydrogen atom or a halogen atom, Z¹ is an oxygen atom, A¹ is a single bond, -CH₂- or -CH(CH₃)-, and G¹ is a C3-C6 cycloalkyl group optionally substituted with at least one member selected from the group consisting of a halogen atom, a C1-C4 alkyl group and a hydroxyl group;
an amide compound of the formula (1), wherein X¹ is a fluorine atom, X² is a fluorine atom, Z¹ is an oxygen atom, A¹ is a single bond, -CH₂- or -CH(CH₃)-, and G¹ is a C3-C6 cycloalkyl group optionally substituted with at least one member selected from the group consisting of a halogen atom, a C1-C4 alkyl group and a hydroxyl group;
an amide compound of the formula (1), wherein X¹ is a fluorine atom, Z¹ is an oxygen atom, and A¹-G¹ is 2-methylcyclohexyl group;
an amide compound of the formula (1), wherein X¹ is a fluorine atom, Z¹ is an oxygen atom, and A¹-G¹ is a 2-chlorocyclohexyl group;
an amide compound of the formula (1), wherein X¹ is a fluorine atom, Z¹ is an oxygen atom, and A¹-G¹ is a cyclohexylmethyl group;
an amide compound of the formula (1), wherein X¹ is a fluorine atom, Z¹ is an oxygen atom, and A¹-G¹ is a 1-cyclohexylethyl group;
an amide compound of the formula (1), wherein X¹ is a fluorine atom, Z¹ is an oxygen atom, and A¹-G¹ is a (1-hydroxycyclohexyl)methyl group;
an amide compound of the formula (1), wherein X¹ is a fluorine atom, Z¹ is an oxygen atom, and A¹-G¹ is a 1-(1-hydroxycyclohexyl)ethyl group;
an amide compound of the formula (1), wherein X¹ is a fluorine atom, Z¹ is an oxygen atom, and A¹-G¹ is a cyclobutylmethyl group;
an amide compound of the formula (1), wherein X¹ is a fluorine atom, Z¹ is an oxygen atom, and A¹-G¹ is a 1-cyclobutylethyl group;
an amide compound of the formula (1), wherein X¹ is a fluorine atom, Z¹ is an oxygen atom, and A¹-G¹ is a (1-hydroxycyclobutyl)methyl group; and
an amide compound of the formula (1), wherein X¹ is a fluorine atom, Z¹ is an oxygen atom, and A¹-G¹ is 1-(1-hydroxycyclobutyl)ethyl group.

The amide compound contained in the plant disease control agent of the present invention is preferably an amide compound of the formula (1) wherein A¹ is a single bond, -CH₂-, -CH(CH₃)-, -C(CH₃)₂-, -CH(CH₂CH₃)- or a substituted methylene, G¹ is a C3-C6 cycloalkyl group which may be substituted with a member of the group [a-1], a C3-C6 cycloalkenyl group which may be substituted with a member of the group [a-1], a substituted cycloalkyl group, or a C3-C6 hydroxyiminocycloalkyl group which may be substituted with a member of the group [a-1], and more preferably an amide compound of the formula (1) wherein X² is a hydrogen atom or a halogen atom and G¹ is a C3-C6 cycloalkyl group optionally substituted with at least one member selected from the group consisting of a halogen atom, a C1-C4 alkyl group and a hydroxyl group.

Examples of the amide compound contained in the plant disease control agent of the present invention further include the following aspects:
an amide compound of the formula (1), wherein A¹ is -CH₂-, -CH(CH₃)-, or methylene substituted with a C2-C5 alkoxycarbonyl group, and G¹ is a CR⁶R⁷R⁸ group, in which R⁶, R⁷ and R⁸ are as defined above;
an amide compound of the formula (1), wherein A¹ is -CH₂-, -CH(CH₃)- or -C(CH₃)₂-, and G¹ is a CR⁶R⁷R⁸ group, in which R⁶, R⁷ and R⁸ are as defined above;
an amide compound of the formula (1), wherein A¹ is -C(CH₃)₂-, and G¹ is a CR⁶R⁷R⁸ group, in which R⁶, R⁷ and R⁸ are as defined above;
an amide compound of the formula (1), wherein G¹ is a CR⁶R⁷R⁸ group, R⁶ is a methyl group, R⁷ is a methyl group, and R⁸ is as defined above;
an amide compound of the formula (1), wherein A¹ is -CH₂-, -CH(CH₃)- or -C(CH₃)₂-, G¹ is a CR⁶R⁷R⁸ group, R⁶ is a methyl group, R⁷ is a methyl group, and R⁸ is as defined above;
an amide compound of the formula (1), wherein A¹ is -CH₂-, G¹ is a CR⁶R⁷R⁸ group, R⁶ is a methyl group, R⁷ is a methyl group, and R⁸ is as defined above;
an amide compound of the formula (1), wherein A¹ is -CH(CH₃)-, G¹ is a CR⁶R⁷R⁸ group, R⁶ is a methyl group, R⁷ is a methyl group, and R⁸ is as defined above;
an amide compound of the formula (1), wherein A¹ is -C(CH₃)₂-, G¹ is a CR⁶R⁷R⁸ group, R⁶ is a methyl group, R⁷ is a methyl group, and R⁸ is as defined above;
an amide compound of the formula (1), wherein A¹ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group, and G¹ is a CR⁶R⁷R⁸ group, in which R⁶, R⁷ and R⁸ are as defined above;

an amide compound of the formula (1), wherein X¹ is a fluorine atom, A¹ is -CH₂-, -CH(CH₃)- or -C(CH₃)₂-, and G¹ is a CR⁶R⁷R⁸ group, in which R⁶, R⁷ and R⁸ are as defined above;
an amide compound of the formula (1), wherein A¹ is -CH₂-, and G¹ is a CR⁶R⁷R⁸ group, in which R⁶, R⁷ and R⁸ are as defined above;
an amide compound of the formula (1), wherein X¹ is a fluorine atom, A¹ is -CH(CH₃)-, and G¹ is CR⁶R⁷R⁸ group, in which R⁶, R⁷ and R⁸ are as defined above;
an amide compound of the formula (1), wherein X¹ is a fluorine atom, A¹ is -C(CH₃)₂-, and G¹ is a CR⁶R⁷R⁸ group, in which R⁶, R⁷ and R⁸ are as defined above;
an amide compound of the formula (1), wherein X¹ is a fluorine atom, G¹ is a CR⁶R⁷R⁸ group, R⁶ is a methyl group, R⁷ is a methyl group, and R⁸ is as defined above;
an amide compound of the formula (1), wherein X¹ is a fluorine atom, A¹ is -CH₂-, -CH(CH₃)- or -C(CH₃)₂-, G¹ is a CR⁶R⁷R⁸ group, R⁶ is a methyl group, R⁷ is a methyl group, and R⁸ is as defined above;
an amide compound of the formula (1), wherein X¹ is a fluorine atom, A¹ is a CH₂ group, G¹ is a CR⁶R⁷R⁸ group, R⁶ is a methyl group, R⁷ is a methyl group, and R⁸ is as defined above;
an amide compound of the formula (1), wherein X¹ is a fluorine atom, A¹ is -CH(CH₃)-, G¹ is a CR⁶R⁷R⁸ group, R⁶ is a methyl group, R⁷ is a methyl group, and R⁸ is as defined above;
an amide compound of the formula (1), wherein X¹ is a fluorine atom, A¹ is -C(CH₃)₂-, G¹ is a CR⁶R⁷R⁸ group, R⁶ is a methyl group, R⁷ is a methyl group, and R⁸ is as defined above;
an amide compound of the formula (1), wherein X¹ is a fluorine atom, G¹ is a CR⁶R⁷R⁸ group, and A¹-CR⁶R⁷R⁸ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group;

an amide compound of the formula (1), wherein X² is a hydrogen atom or a halogen atom, A¹ is -CH₂-, -CH(CH₃)-, or methylene substituted with a C2-C5 alkoxycarbonyl group, and G¹ is a CR⁶R⁷R⁸ group, in which R⁶, R⁷ and R⁸ are as defined above;
an amide compound of the formula (1), wherein X² is a hydrogen atom or a fluorine atom, A¹ is -CH₂-, -CH(CH₃)- or -C(CH₃)₂-, and G¹ is a CR⁶R⁷R⁸ group, in which R⁶, R⁷ and R⁸ are as defined above;
an amide compound of the formula (1), wherein X² is a hydrogen atom or a fluorine atom, A¹ is -CH₂-, and G¹ is a CR⁶R⁷R⁸ group, in which R⁶, R⁷ and R⁸ are as defined above; an amide compound of the formula (1), wherein X² is a hydrogen atom or a fluorine atom, A¹ is -CH(CH₃)-, and G¹ is a CR⁶R⁷R⁸ group, in which R⁶, R⁷ and R⁸ are as defined above;
an amide compound of the formula (1), wherein X² is a hydrogen atom or a fluorine atom, A¹ is -C(CH₃)₂-, and G¹ is a CR⁶R⁷R⁸ group, in which R⁶, R⁷ and R⁸ are as defined above;
an amide compound of the formula (1), wherein X² is a hydrogen atom or a fluorine atom, G¹ is a CR⁶R⁷R⁸ group, R⁶ is a methyl group, R⁷ is a methyl group, and R⁸ is as defined above;
an amide compound of the formula (1), wherein X² is a hydrogen atom or a fluorine atom, A¹ is -CH₂-, -CH(CH₃)- or -C(CH₃)₂-, G¹ is a CR⁶R⁷R⁸ group, R⁶ is a methyl group, R⁷ is a methyl group, and R⁸ is as defined above;
an amide compound of the formula (1), wherein X² is a hydrogen atom or a fluorine atom, A¹ is -CH₂-, G¹ is a CR⁶R⁷R⁸ group, R⁶ is a methyl group, R⁷ is a methyl group, and R⁸ is as defined above;
an amide compound of the formula (1), wherein X² is a hydrogen atom or a fluorine atom, A¹ is -CH(CH₃)-, G¹ is a CR⁶R⁷R⁸ group, R⁶ is a methyl group, R⁷ is a methyl group, and R⁸ is as defined above;
an amide compound of the formula (1), wherein X² is a hydrogen atom or a fluorine atom, A¹ is -C(CH₃)₂-, G¹ is a CR⁶R⁷R⁸ group, R⁶ is a methyl group, R⁷ is a methyl group, and R⁸ is as defined above;
an amide compound of the formula (1), wherein X² is a hydrogen atom or a fluorine atom, G¹ is a CR⁶R⁷R⁸ group, and A¹-CR⁶R⁷R⁸ is a 2-methylpropyl group, a 1,2-dimethylpropyl group, a 2,2-dimethylpropyl group, a 1,2,2-trimethylpropyl group, a 2-methyl-2-hydroxypropyl group or a 1,2-dimethyl-2-hydroxypropyl group;

an amide compound of the formula (1), wherein X¹ is a fluorine atom, X² is a hydrogen atom or a fluorine atom, A¹ is -CH₂-, -CH(CH₃)- or -C(CH₃)₂-, and G¹ is a CR⁶R⁷R⁸ group, in which R⁶, R⁷ and R⁸ are as defined above;
an amide compound of the formula (1), wherein X¹ is a fluorine atom, X² is a hydrogen atom or a fluorine atom, A¹ is -CH₂-, and G¹ is a CR⁶R⁷R⁸ group, in which R⁶, R⁷ and R⁸ are as defined above;
an amide compound of the formula (1), wherein X¹ is a fluorine atom, X² is a hydrogen atom or a fluorine atom, A¹ is -CH(CH₃)-, and G¹ is a CR⁶R⁷R⁸ group, in which R⁶, R⁷ and R⁸ are as defined above;
an amide compound of the formula (1), wherein X¹ is a fluorine atom, X² is a hydrogen atom or a fluorine atom, A¹ is -C(CH₃)₂-, and G¹ is a CR⁶R⁷R⁸ group, in which R⁶, R⁷ and R⁸ are as defined above;
an amide compound of the formula (1), wherein X¹ is a fluorine atom, X² is a hydrogen atom or a fluorine atom, G¹ is a CR⁶R⁷R⁸ group, R⁶ is a methyl group, R⁷ is a methyl group, and R⁸ is as defined above;
an amide compound of the formula (1), wherein X¹ is a fluorine atom, X² is a hydrogen atom or a fluorine atom, A¹ is -CH₂-, -CH(CH₃)- or -C(CH₃)₂-, G¹ is a CR⁶R⁷R⁸ group, R⁶ is a methyl group, R⁷ is a methyl group, and R⁸ is as defined above;
an amide compound of the formula (1), wherein X¹ is a fluorine atom, X² is a hydrogen atom or a fluorine atom, A¹ is -CH₂-, G¹ is a CR⁶R⁷R⁸ group, R⁶ is a methyl group, R⁷ is a methyl group, and R⁸ is as defined above;
an amide compound of the formula (1), wherein X¹ is a fluorine atom, X² is a hydrogen atom or a fluorine atom, A¹ is -CH(CH₃)-, G¹ is a CR⁶R⁷R⁸ group, R⁶ is a methyl group, R⁷ is a methyl group, and R⁸ is as defined above;
an amide compound of the formula (1), wherein X¹ is a fluorine atom, X² is a hydrogen atom or a fluorine atom, A¹ is -C(CH₃)₂-, G¹ is a CR⁶R⁷R⁸ group, R⁶ is a methyl group, R⁷ is a methyl group, and R⁸ is as defined above;
an amide compound of the formula (1), wherein X² is a hydrogen atom or a halogen atom, A¹ is -CH₂-, -CH(CH₃)-, or methylene substitute with a C2-C5 alkoxycarbonyl group, G¹ is a CR⁶R⁷R⁸ group, R⁶ and R⁷ are as defined above, and R⁸ is a hydrogen atom or a C1-C4 alkyl group;
an amide compound of the formula (1), wherein X² is a hydrogen atom or a halogen atom, Z¹ is an oxygen atom, A¹ is -CH₂-, -CH(CH₃)-, or methylene substituted with a C2-C5 alkoxycarbonyl group, G¹ is a CR⁶R⁷R⁸ group, R⁶ and R⁷ are as defined above, and R⁸ is a hydrogen atom or a C1-C4 alkyl group;
an amide compound of the formula (1), wherein X¹ is a fluorine atom, X² is a hydrogen atom or a fluorine atom, A¹ is -CH₂-, -CH(CH₃)-, or methylene substituted with a C2-C5 alkoxycarbonyl group, G¹ is a CR⁶R⁷R⁸ group, R⁶ and R⁷ are as defined above, and R⁸ is a hydrogen atom or a C1-C4 alkyl group; and
an amide compound of the formula (1), wherein X¹ is a fluorine atom, X² is a hydrogen atom or a fluorine atom, Z¹ is an oxygen atom, A¹ is -CH₂-, -CH(CH₃)-, or methylene substituted with a C2-C5 alkoxycarbonyl group, G¹ is a CR⁶R⁷R⁸ group, R⁶ and R⁷ are as defined above, and R⁸ is a hydrogen atom or a C1-C4 alkyl group;

an amide compound of the formula (1), wherein A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group and a C2-C4 alkynyl group,
G¹ is a CR⁶R⁷R¹¹ group (provided that A¹ is not a single bond when G¹ is a CR⁶R⁷R¹¹ group), a C3-C6 cycloalkyl group optionally substituted with at least one member selected from the group [a-3] shown below, a 3-C6 cycloalkenyl group optionally substituted with at least one member selected from the group [a-3] shown below, or a C3-C6 cycloalkyl group optionally substituted with at least one member selected from the group [a-3] shown below wherein one methylene forming ring is replaced by a carbonyl group,
R⁶ and R⁷ are as defined above,
R¹¹ is a hydrogen atom, a halogen atom, a C1-C4 alkyl group, a C2-C4 alkenyl group, a C2-C4 alkynyl group, a hydroxyl group, a C1-C6 alkoxy group, a C3-C6 alkenyloxy group, a C1-C6 haloalkyl group, a C1-C6 haloalkoxy group, a C1-C3 alkylthio group, a C1-C6 hydroxyalkyl group or a C2-C4 alkylcarbonyloxy group, and
the group [a-3] consists of:
a halogen atom, a C1-C4 alkyl group, a C2-C4 alkenyl group, a C2-C4 alkynyl group, a hydroxyl group, a C1-C6 alkoxy group, a C3-C6 alkenyloxy group, a C1-C6 haloalkyl group, a C1-C6 haloalkoxy group, a C1-C3 alkylthio group, a C1-C3 alkylidene group which forms a double bond with a ring-forming carbon atom, a C1-C6 hydroxyalkyl group, and a C2-C4 alkylcarbonyloxy group; and

an amide compound of the formula (1), wherein A¹ is a single bond, -CH₂- or -CH(CH₃)-,
G¹ is a CR⁶R⁷R¹¹ group (provided that A¹ is not a single bond when G¹ is a CR⁶R⁷R¹¹ group), a C3-C6 cycloalkyl group optionally substituted with at least one member selected from the group [a-3], a C3-C6 cycloalkenyl group optionally substituted with at least one member selected from the group [a-3], or a C3-C6 cycloalkyl group optionally substituted with at least one member selected from the group [a-3] wherein one methylene forming ring is replaced by a carbonyl group, and
R⁶, R⁷ and R¹¹ are as defined above.

The amide compound contained in the plant disease control agent of the present invention is preferably an amide compound of the formula (1) wherein A¹ is -CH₂-, -CH(CH₃)-, -C(CH₃)₂-, -CH(CH₂CH₃)- or a substituted methylene when G¹ is a CR⁶R⁷R⁸ group, and more preferably an amide compound of the formula (1) wherein X² is a hydrogen atom or a halogen atom, A¹ is -CH₂-, -CH(CH₃)- or methylene substituted with a C2-C5 alkoxycarbonyl group, and R⁸ is a hydrogen atom or a C1-C4 alkyl group.

A particularly preferable example of the amide compound represented by the above formula (1) is a 3,5-difluoro-4-methoxybenzamide compound represented by the formula (A) shown below. The 3,5-difluoro-4-methoxybenzamide compound represented by the formula (A) shown below is also an aspect of the present invention.

Formula (A):

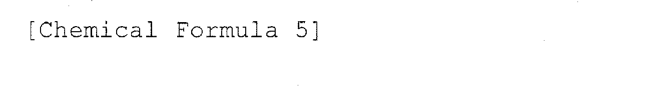

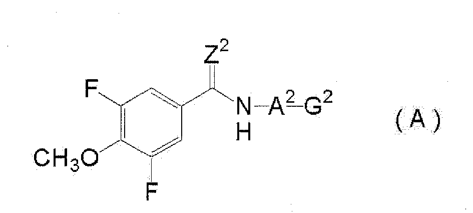

In the formula (A), Z² represents an oxygen atom or a sulfur atom.
In the formula (A), A² represents a single bond, -CH₂-, -CH(CH₃)-, -C(CH₃)₂-, -CH(CH₂CH₃)-, or a substituted methylene.
In the formula (A), G² is a CR²⁶R²⁷R²⁸ group (provided that A² is not a single bond when G² is a CR²⁶R²⁷R²⁸ group), a C3-C6 cycloalkyl group which may be substituted with a member of the group [a-2], a C3-C6 cycloalkenyl group which may be substituted with a member of the group [a-2], a C3-C6 cycloalkyl group which may be substituted with a member of the group [a-2] wherein one methylene forming ring is replaced by a carbonyl group, or a C3-C6 hydroxyiminocycloalkyl group which may be substituted with a member of the group [a-2].
The phrase "a group of the group [a-2]" means "at least one member selected from the group [a-2]".

R²⁶ and R²⁷ independently represent a C1-C4 alkyl group.
R²⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group, or an NR²⁹R³⁰ group in which R²⁹ and R³⁰ independently represent a hydrogen atom, a C1-C4 alkyl group, a C2-C5 alkylcarbonyl group, a C2-C5 alkoxycarbonyl group or a C1-C4 alkylsulfonyl group.

The group [a-2] consists of the following atoms and groups:
a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group, and an NR²⁹R³⁰ group in which R²⁹ and R³⁰ are as defined above.

Examples of the substituted methylene represented by A² are the same as those of the substituted methylene represented by A¹.
Examples of the substituent represented by R²⁶ are the same as those of the substituent represented by R⁶ Examples of the substituent represented by R²⁷ are the same as those of the substituent represented by R⁷. Examples of the substituent represented by R²⁸ are the same as those of the substituent represented by R⁸. Examples of the substituent represented by the group [a-2] are the same as those of the substituent represented by the group [a-1].

The 3,5-difluoro-4-methoxybenzamide compound of the present invention is preferably a 3,5-difluoro-4-methoxybenzamide compound of the formula (A) wherein G² is a C3-C6 cycloalkyl group which may be substituted with a member of the group [a-2], a C3-C6 cycloalkenyl group which may be substituted with a member of the group [a-2], a C3-C6 cycloalkyl group which may be substituted with a member of the group [a-2] wherein one methylene forming ring is replaced by a carbonyl group, or a C3-C6 hydroxyiminocycloalkyl group which may be substituted with a member of the group [a-2], and more preferably a 3,5-difluoro-4-methoxybenzamide compound of the formula (A) wherein G² is a C3-C6 cycloalkyl group optionally substituted with at least one member selected from the group consisting of a halogen atom, a C1-C4 alkyl group and a hydroxyl group.

The 3,5-difluoro-4-methoxybenzamide compound of the present invention is preferably a 3,5-difluoro-4-methoxybenzamide compound of the formula (A) wherein A² is -CH₂ -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group when G² is a CR²⁶R²⁷R²⁸ group in which R²⁶, R²⁷ and R²⁸ are as defined above, more preferably a 3,5-difluoro-4-methoxybenzamide compound of the formula (A) wherein R²⁸ is a hydrogen atom or a C1-C4 alkyl group, and still more preferably a 3,5-difluoro-4-methoxybenzamide compound of the formula (A) wherein Z² is an oxygen atom and A² is -CH(CH₃)-.

Examples of the 3,5-difluoro-4-methoxybenzamide compound of the present invention further includes the following compounds.
An amide compound of the formula (A), wherein A² is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group and a C2-C4 alkynyl group, G² is a CR²⁶R²⁷R³¹ group (provided that A² is not a single bond when G² is a CR²⁶R²⁷R³¹ group), a C3-C6 cycloalkyl group optionally substituted with at least one member selected from the group [a-3], a C3-C6 cycloalkenyl group optionally substituted with at least one member selected from the group [a-3], or a C3-C6 cycloalkyl group optionally substituted with at least one member selected from the group [a-3] wherein one methylene forming ring is replaced by a carbonyl group, R²⁶ and R²⁷ are as defined above, and
R³¹ is a hydrogen atom, a halogen atom, a C1-C4 alkyl group, a C2-C4 alkenyl group, a C2-C4 alkynyl group, a hydroxyl group, a C1-C6 alkoxy group, a C3-C6 alkenyloxy group, a C1-C6 haloalkyl group, a C1-C6 haloalkoxy group, a C1-C3 alkylthio group, a C1-C6 hydroxyalkyl group or a C2-C4 alkylcarbonyloxy group.

An amide compound of the formula (A), wherein A² is a single bond, -CH₂- or -CH(CH₃)-.
G² is a CR²⁶R²⁷R³¹ group (provided that A² is not a single bond when G² is a CR²⁶R²⁷R³¹ group), a C3-C6 cycloalkyl group optionally substituted with at least one member selected from the group [a-3], a C3-C6 cycloalkenyl group optionally substituted with at least one member selected from the group [a-3], or a C3-C6 cycloalkyl group optionally substituted with at least one member selected from the group [a-3] wherein one methylene forming ring is replaced by a carbonyl group, and
R²⁶, R²⁷ and R³¹ are as defined above.

The amide compound represented the formula (1) can be produced, for example, by Synthesis Process 1 to Synthesis Process 9 shown below.

### (Synthesis Process 1)

Among amide compounds represented by the formula (1), a compound (5) wherein Z¹ is an oxygen atom can be produced by reacting a compound (2) or a salt thereof with a compound (3) in the presence of a dehydration condensing agent: 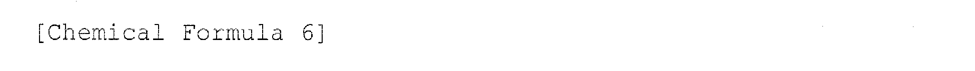 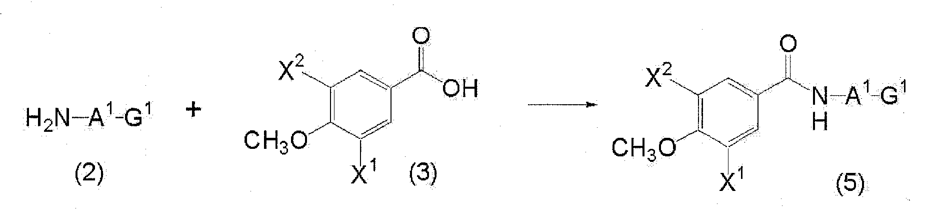 wherein A¹, G¹, X¹ and X² are as defined above.
The reaction is usually carried out in the presence of a solvent.
Examples of the solvent used for the reaction include ethers such as tetrahydrofuran (hereinafter, may be referred to as THF), ethylene glycol dimethyl ether and tert-butyl methyl ether (hereinafter, may be referred to as MTBE); aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as N,N-dimethyl formamide (hereinafter, may be referred to as DMF); sulfoxides such as dimethyl sulfoxide (hereinafter, may be referred to as DMSO); and their mixtures.
Examples of the dehydration condensing agent used for the reaction include carbodiimides such as 1-ethyl-3-(3-dimethylaminopropyl)carbodiimide hydrochloride (hereinafter, may be referred to as WSC) and 1,3-dicyclohexylcarbodiimide; and phosphonium salts such as (benzotriazol-1-yloxy)tris(dimethylamino)phosphonium hexafluorophosphate (hereinafter, referred to as a BOP reagent).
The used amount of the compound (3) is usually 1 to 3 mol per 1 mol of the compound (2) or its salt, and the used amount of the dehydration condensing agent is usually 1 to 5 mol per 1 mol of the compound (2) or its salt.
The reaction temperature is usually within a range from 0 to 140°C, and the reaction time is usually within a range from 1 to 24 hours.
After completion of the reaction, the compound (5) can be isolated by post-treatment such as filtration of the reaction mixture, extraction of the filtrate with an organic solvent, and drying and concentration of an organic layer. The isolated compound (5) can be further purified by chromatography, recrystallization and so on.

### (Synthesis Process 2)

Among amide compounds represented by the formula (1), the compound (5) wherein Z¹ is an oxygen atom can be produced by reacting the compound (2) or a salt thereof with a compound (4) in the presence of a base: 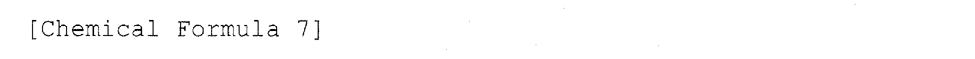 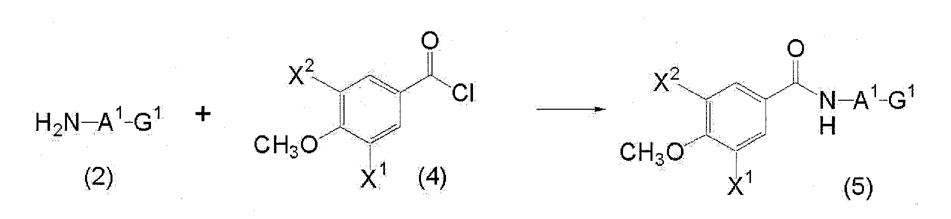 wherein A¹, G¹, X¹ and X² are as defined above.
The reaction is usually carried out in the presence of a solvent.
Examples of the solvent used for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as DMF; sulfoxides such as DMSO; and their mixtures.
Examples of the base used for the reaction include alkali metal carbonates such as sodium carbonate and potassium carbonate; tertiary amines such as triethylamine and diisopropylethylamine; and nitrogen-containing aromatic compounds such as pyridine and 4-dimethylaminopyridine.
The used amount of the compound (4) is usually 1 to 3 mol per 1 mol of the compound (2) or a salt thereof, and the used amount of the base is usually 1 to 10 mol per 1 mol of the compound (2) or a salt thereof.
The reaction temperature is usually within a range from -20 to 100°C, and the reaction time is usually within a range from 0.1 to 24 hours.
After completion of the reaction, the compound (5) can be isolated by post-treatment such as extraction of the reaction mixture with an organic solvent, and drying and concentration of an organic layer. The isolated compound (5) can be further purified by chromatography, recrystallization and so on.

### (Synthesis Process 3)

Among amide compounds represented by the formula (1), a compound (6) wherein Z¹ is a sulfur atom can be produced by reacting the compound (5) wherein Z¹ is an oxygen atom among amide compounds of the formula (1) with sulfurizing agent such as 2,4-bis(4-methoxyphenyl)-1,3-dithia-2,4-diphosphethane-2,4-disulfide (hereinafter, referred to as Lawesson's reagent) or a phosphorous pentasulphide: 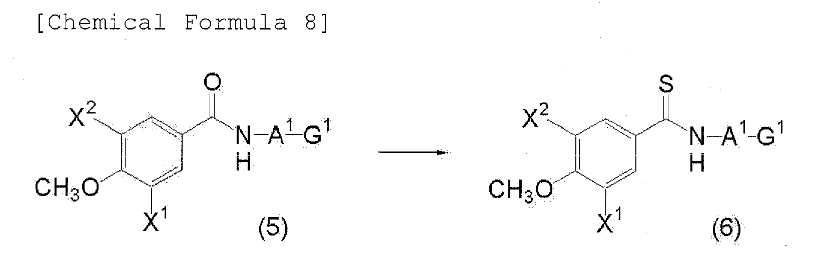 wherein A¹, G¹, X¹ and X² are as defined above.
The reaction is usually carried out in the presence of a solvent.
Examples of the solvent used for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; organic nitriles such as acetonitrile and butyronitrile; sulfoxides such as dimethyl sulfoxide; and their mixtures.
The used amount of the sulfurizing agent is usually 1 to 2 mol per 1 mol of the compound (5).
The reaction temperature is usually within a range from 25 to 150°C, and the reaction time is usually within a range from 0.1 to 24 hours.
After completion of the reaction, the compound (6) can be isolated by post-treatment such as extraction of the reaction mixture with an organic solvent, and drying and concentration of an organic layer. The isolated compound (6) can be further purified by chromatography, recrystallization and so on.

### (Synthesis Process 4)

Among amide compounds represented by the formula (1), a compound (9) wherein Z¹ is an oxygen atom and X¹ is a fluorine atom can be produced by reacting a compound (7) with the compound (2) in the presence of a base to obtain a compound (8) (step (IV-1)) and then reacting the compound (8) with methanol in the presence of a base (step (IV-2)): 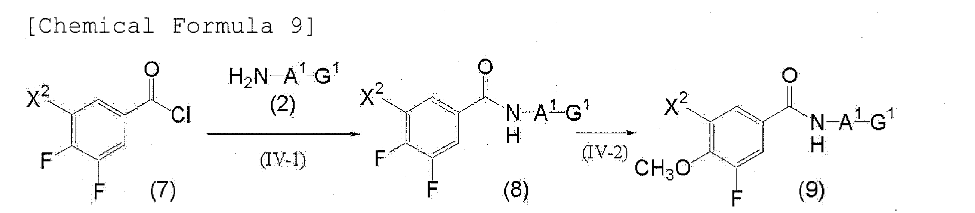 wherein A¹, G¹ and X² are as defined above.

### Step (IV-1)

The reaction is usually carried out in the presence of a solvent.

Examples of the solvent used for the reaction include ethers such as THF, ethylene glycol dimethyl ether and MTBE; aliphatic hydrocarbons such as hexane, heptane and octane; aromatic hydrocarbons such as toluene and xylene; halogenated hydrocarbons such as chlorobenzene; esters such as butyl acetate and ethyl acetate; nitriles such as acetonitrile; acid amides such as DMF; sulfoxides such as dimethyl sulfoxide; and their mixtures.

Examples of the base used for the reaction include alkali metal carbonates such as sodium carbonate and potassium carbonate; tertiary amines such as triethylamine and diisopropylethylamine; and nitrogen-containing aromatic compounds such as pyridine and 4-dimethylaminopyridine.

The used amount of the compound (7) is usually 1 to 3 mol per 1 mol of the compound (2), and the used amount of the base is usually 1 to 10 mol per 1 mol of the compound (2).

The reaction temperature is usually within a range from -20 to 100°C, and the reaction time is usually within a range from 0.1 to 24 hours.

After completion of the reaction, the compound (8) can be isolated by post-treatment such as extraction of the reaction mixture with an organic solvent, and drying and concentration of an organic layer. The isolated compound (8) can be further purified by chromatography, recrystallization and so on.

### Step (IV-2)

The reaction is usually carried out in the presence of a solvent.

Examples of the solvent used for the reaction include ethers such as THF, ethylene glycol dimethyl ether and MTBE; aliphatic hydrocarbons such as hexane, heptane and octane; ketones such as acetone, methyl ethyl ketone and methyl isobutyl ketone; aromatic hydrocarbons such as toluene and xylene; halogenated hydrocarbons such as chlorobenzene; esters such as butyl acetate and ethyl acetate; nitriles such as acetonitrile; acid amides such as DMF; sulfoxides such as dimethyl sulfoxide; water; and their mixtures.

Examples of the base used for the reaction include alkali metal carbonates such as sodium carbonate and potassium carbonate; alkali metal hydrogen carbonates such as sodium hydrogen carbonate; alkali metal hydrides such as sodium hydride; and alkali metal hydroxides such as sodium hydroxide.

The used amount of methanol is usually 1 mol to an excess amount per 1 mol of the compound (8), and the used amount of the base is usually 1 to 2 mol per 1 mol of the compound (8).

The reaction temperature is usually within a range from -20 to 150°C, and the reaction time is usually within a range from 0.1 to 24 hours.

After completion of the reaction, the compound (9) can be isolated by post-treatment such as extraction of the reaction mixture with an organic solvent, and drying and concentration of an organic layer. The isolated compound (9) can be further purified by chromatography, recrystallization and so on.

### (Synthesis Process 5)

Among amide compounds represented by the formula (1), the compound (5) wherein Z¹ is an oxygen atom can be produced by reacting a compound (10) with a methylating agent such as methyl iodide or dimethylsulfuric acid in the presence of a base: 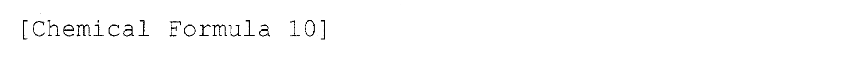 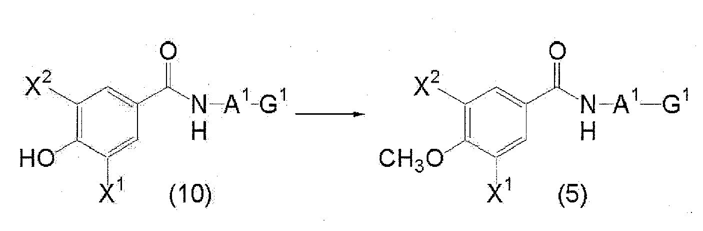 wherein A¹, G¹, X¹ and X² are as defined above.

The reaction is usually carried out in the presence of a solvent.

Examples of the solvent used for the reaction include ethers such as THF, ethylene glycol dimethyl ether and MTBE; aromatic hydrocarbons such as toluene and xylene; halogenated hydrocarbons such as chlorobenzene; nitriles such as acetonitrile; acid amides such as DMF; sulfoxides such as dimethyl sulfoxide; ketones such as acetone, methyl ethyl ketone and methyl isobutyl ketone; water; and their mixtures.

Examples of the base used for the reaction include alkali metal carbonates such as sodium carbonate, potassium carbonate and cesium carbonate; alkali metal hydroxides such as sodium hydroxide; and alkali metal hydrides such as sodium hydride.

The used amount of the methylating agent is usually 1 to 3 mol per 1 mol of the compound (10), and the used amount of the base is usually 1 to 3 mol per 1 mol of the compound (10).

The reaction temperature is usually within a range from -20 to 100°C, and the reaction time is usually within a range from 0.1 to 24 hours.

After completion of the reaction, the compound (5) can be isolated by post-treatment such as extraction of the reaction mixture with an organic solvent, and drying and concentration of an organic layer. The isolated compound (5) can be further purified by chromatography, recrystallization and so on.

### (Synthesis Process 6)

Among amide compounds represented by the formula (1), a compound (12) can be produced by a method shown in the following scheme.

A compound (12) wherein R⁸ is a chlorine atom can be produced by reacting the compound (4) with 7-azabicyclo[4.1.0]heptane without isolating an intermediate (11) : 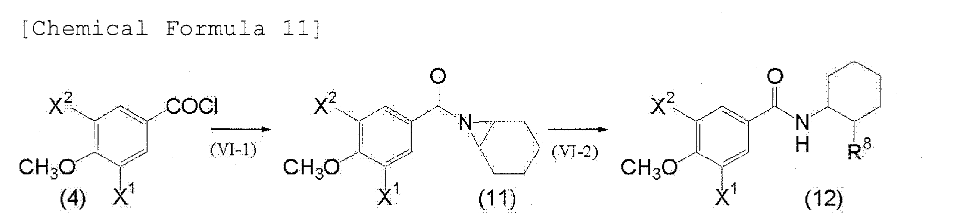 wherein X¹ and X² are as defined above, and R⁸ represents a fluorine atom, a chlorine atom, a bromine atom, an iodine atom, a cyano group, a C1-C3 alkylthio group, a C1-C6 alkoxy group or a phenoxy group.

### Step (VI-1)

The compound (11) can be produced by reacting the compound (4) with 7-azabicyclo[4.1.0]heptane in the presence of a base according to the method described in Production Process 2.

### Step (VI-2)

The compound (12) can be produced by reacting the compound (11) with a reagent described below.

In the case of the compound (12) wherein R⁸ is a fluorine atom, examples of the reagent used for the reaction include alkali metal fluorides such as potassium fluoride and lithium fluoride; alkali earth metal fluorides such as calcium fluoride; quaternary ammonium fluorides such as tetrabutylammonium fluoride; and hydrogen fluoride.

In the case of the compound (12) wherein R⁸ is a chlorine atom, examples of the reagent used for the reaction include alkali metal chlorides such as sodium chloride and lithium chloride; alkali earth metal chlorides such as magnesium chloride; metal chlorides such as aluminum chloride and zinc (II) chloride; quaternary ammonium chlorides such as tetrabutylammonium chloride; organic silicon chlorides such as trimethylsilyl chloride; sulfur compounds such as thionyl chloride; phosphorus compounds such as phosphorus oxychloride, phosphorus trichloride and phosphorus pentachloride; and hydrogen chloride.

In the case of the compound (12) wherein R⁸ is a bromine atom, examples of the reagent used for the reaction include alkali metal bromides such as sodium bromide; alkali earth metal bromides such as magnesium bromide; metal bromides such as zinc (II) bromide; quaternary ammonium bromides such as tetrabutylammonium bromide; organic silicon bromides such as trimethylsilyl bromide; phosphorus compounds such as phosphorus tribromide; and hydrogen bromide.

In the case of the compound (12) wherein R⁸ is an iodine atom, examples of the reagent used for the reaction include alkali metal iodides such as potassium iodide; alkali earth metal iodides such as magnesium iodide; metal iodides such as zinc (II) iodide; quaternary ammonium iodides such as tetrabutylammonium iodide; organic silicon compounds such as trimethylsilyl iodide; and hydrogen iodide. In the case of the compound (12) wherein R⁸ is a cyano group, examples of the reagent used for the reaction include cyanides such as potassium cyanide and sodium cyanide; and organic silicon compounds such as trimethylsilyl cyanide.

In the case of the compound (12) wherein R⁸ is a C1-C3 alkylthio group, examples of the reagent used for the reaction include a C1-C3 alkyl mercaptan.

In the case of the compound (12) wherein R⁸ is a C1-C6 alkoxy group, examples of the reagent used for the reaction include a C1-C6 alcohol.

In the case of the compound (12) wherein R⁸ is a phenoxy group, examples of the reagent used for the reaction include phenol.

The reaction is usually carried out in the presence of a solvent.

Examples of the solvent used for the reaction include ethers such as THF, ethylene glycol dimethyl ether and MTBE; aromatic hydrocarbons such as toluene and xylene; halogenated hydrocarbons such as chlorobenzene and chloroform; esters such as butyl acetate and ethyl acetate; nitriles such as acetonitrile; acid amides such as DMF; water; and their mixtures.

The used amount of the reagent is usually 1 to 10 mol per 1 mol of the compound (11).

The reaction temperature is usually within a range from -20 to 150°C, and the reaction time is usually within a range from 0.1 to 24 hours.

If necessary, the reaction can also be carried out in the presence of an additive. Examples of the additive include phosphorus compounds such as tributyl phosphine.

The above-mentioned C1-C3 alkyl mercaptan, C1-C6 alcohol or phenol is reacted with an alkali metal hydride (eg. sodium hydride) and so on to prepare an alkali metal, and then the alkali metal can also be used for the reaction.

After completion of the reaction, the compound (12) can be isolated by post-treatment such as extraction of the reaction mixture with an organic solvent, and drying and concentration of an organic layer. The isolated compound (12) can be further purified by chromatography, recrystallization and so on.

### (Synthesis Process 7)

Among amide compounds represented by the formula (1), a compound of the formula (15) can be produced by a method described in the following scheme: 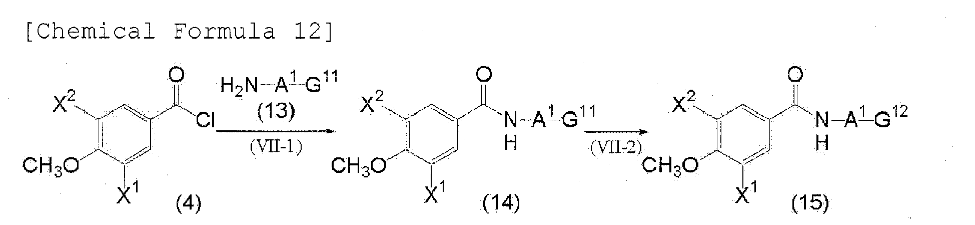 wherein A¹ is as defined above; G¹¹ represents a C3-C6 cycloalkyl group wherein one methylene forming ring is replaced by CH(OH) and which is optionally substituted with at least one member selected from the group consisting of a halogen atom, a C1-C4 alkyl group, a C2-C4 alkenyl group, a C2-C4 alkynyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, benzyl group, a C1-C3 alkylthio group, a C1-C3 alkylidene group which forms a double bond with a ring-forming carbon atom, a C2-C4 alkylcarbonyloxy group, a (C1-C3 alkylamino)C1-C6 alkyl group, a (di(C1-C3 alkyl)amino)C1-C6 alkyl group, a carbamoyl group, a C2-C6 cyanoalkyl group, a C1-C3 alkylsulfonyl group, a phenoxy group and an NR⁶¹R⁷¹ group, in which R⁶¹ and R⁷¹ independently represent a hydrogen atom, a C1-C4 alkyl group, a C2-C5 alkylcarbonyl group, a C2-C5 alkoxycarbonyl group or a C1-C4 alkylsulfonyl group; G¹² represents a C3-C6 cycloalkyl group wherein one methylene forming ring is replaced by a carbonyl group and which is optionally substituted with at least one member selected from the group consisting of a halogen atom, a C1-C4 alkyl group, a C2-C4 alkenyl group, a C2-C4 alkynyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C2-C4 alkylcarbonyloxy group, a (C1-C3 alkylamino)C1-C6 alkyl group, a (di(C1-C3 alkyl)amino)C1-C6 alkyl group, a carbamoyl group, a C2-C6 cyanoalkyl group, a C1-C3 alkylsulfonyl group, a phenoxy group and an NR⁶²R⁷² group, in which R⁶² and R⁷² independently represent a hydrogen atom, a C1-C4 alkyl group, a C2-C5 alkylcarbonyl group, a C2-C5 alkoxycarbonyl group or a C1-C4 alkylsulfonyl group; and X¹ and X² are as defined above.

### Step (VII-1)

The compound (14) can be produced by reacting the compound (4) with the compound (13) in the presence of a base according to the method described in Production Process 2.

### Step (VII-2)

The compound (15) can be produced by reacting the compound (14) with an oxidizing agent.

The reaction is usually carried out in the presence of a solvent.

Examples of the solvent used for the reaction include ketones such as acetone, methyl ethyl ketone and methyl isobutyl ketone; halogenated hydrocarbons such as chloroform; water; and their mixtures.

Examples of the oxidizing agent used for the reaction include hypervalent iodine compounds such as bis(acetoxy)phenyl iodide; chromium compounds such as potassium bichromate and chromic acid; halogen oxide compounds such as periodic acid; and manganese oxides such as manganese dioxide and potassium permanganate.

The used amount of the oxidizing agent is usually 1 to 10 mol per 1 mol of the compound (14).

The reaction temperature is usually within a range from -78 to 150°C, and the reaction time is usually within a range from 0.1 to 24 hours.

After completion of the reaction, the compound (15) can be isolated by post-treatment such as pouring of the reaction mixture into water, extraction with an organic solvent, and drying and concentration of an organic layer. The isolated compound (15) can be further purified by chromatography, recrystallization and so on.

### (Synthesis Process 8)

Among amide compounds represented by the formula (1), an amide compound of the formula (17) can be produced by reacting the compound (15) with the compound (16) in the presence of a base: 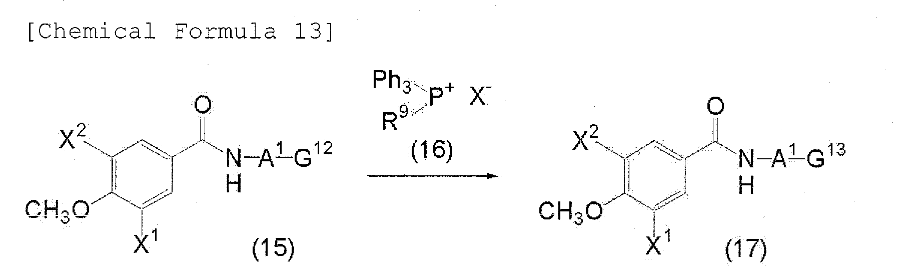 wherein G¹³ represents a C3-C6 cycloalkyl group which is substituted with a C1-C3 alkylidene group which forms a double bond with a ring-forming carbon atom and which is optionally substituted with at least one member selected from the group consisting of a halogen atom, a C1-C4 alkyl group, a C2-C4 alkenyl group, a C2-C4 alkynyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C2-C4 alkylcarbonyloxy group, a (C1-C3 alkylamino)C1-C6 alkyl group, a (di(C1-C3 alkyl)amino)C1-C6 alkyl group, a carbamoyl group, a C2-C6 cyanoalkyl group, a C1-C3 alkylsulfonyl group, a phenoxy group and an NR⁶³R⁷³ group, in which R⁶³ and R⁷³ independently represent a hydrogen atom, a C1-C4 alkyl group, a C2-C5 alkylcarbonyl group, a C2-C5 alkoxycarbonyl group or a C1-C4 alkylsulfonyl group; R⁹ represents a C1-C3 alkyl group; X represents a chlorine atom, a bromine atom or an iodine atom; and X¹, X², A¹ and G¹² are as defined above.

The reaction is usually carried out in the presence of a solvent.

Examples of the solvent used for the reaction include ketones such as acetone, methyl ethyl ketone and methyl isobutyl ketone; halogenated hydrocarbons such as chloroform; water; and tehir mixtures.

Examples of the base used for the reaction include alkali metal salts such as potassium tert-butoxide and n-butyl lithium; and alkali metal hydroxides such as sodium hydroxide.

The used amount of the compound of the formula (16) is usually 1 to 10 mol per 1 mol of the compound (15), and the used amount of the base is usually 1 to 10 mol per 1 mol of the compound (15).

The reaction temperature is usually within a range from -78 to 150°C, and the reaction time is usually within a range from 0.1 to 24 hours.

After completion of the reaction, the compound (17) can be isolated by post-treatment such as extraction of the reaction mixture with an organic solvent, and drying and concentration of an organic layer. The isolated compound (17) can be further purified by chromatography, recrystallization and so on.

### (Synthesis Process 9)

Among amide compounds represented by the formula (1), an amide compound of the formula (18) can be produced by reacting the compound (15) with hydroxylamine or a salt thereof: 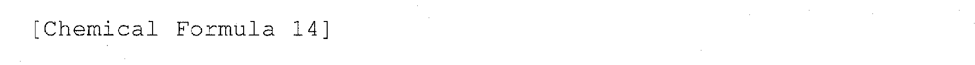 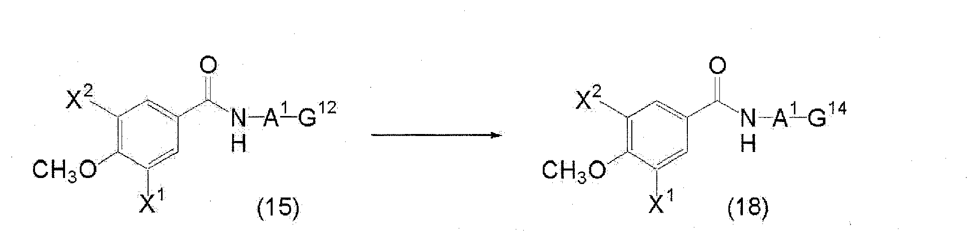 wherein G¹⁴ represents C3-C6 hydroxyiminocycloalkyl group which is optionally substituted with at least one member selected from the group consisting of a halogen atom, a C1-C4 alkyl group, a C2-C4 alkenyl group, a C2-C4 alkynyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C2-C4 alkylcarbonyloxy group, a (C1-C3 alkylamino)Cl-C6 alkyl group, a (di(C1-C3 alkyl)amino)C1-C6 alkyl group, a carbamoyl group, a C2-C6 cyanoalkyl group, a C1-C3 alkylsulfonyl group, a phenoxy group and an NR⁶⁴R⁷⁴ group, in which R⁶⁴ and R⁷⁴ independently represent a hydrogen atom, a C1-C4 alkyl group, a C2-C5 alkylcarbonyl group, a C2-C5 alkoxycarbonyl group or a C1-C4 alkylsulfonyl group; and A¹, A³, X¹, X² and G¹² are as defined above.

The reaction is usually carried out in the presence of a solvent.

Examples of the solvent used for the reaction include ethers such as 1,4-dioxane, THF and MTBE; aliphatic hydrocarbons such as hexane and heptane; aromatic hydrocarbons such as toluene and xylene; halogenated hydrocarbons such as chlorobenzene; esters such as ethyl acetate; nitriles such as acetonitrile and butyronitrile; acid amides such as DMF; sulfoxides such as dimethyl sulfoxide; alcohols such as methanol and ethanol; water; and their mixtures.

The used amount of hydroxylamine or a salt thereof is usually 1 to 5 mol per 1 mol of the compound (15).

The reaction temperature is usually within a range from 0 to 150°C, and the reaction time is usually within a range from 0.1 to 24 hours.

After completion of the reaction, the compound (18) can be isolated by post-treatment such as extraction of the reaction mixture with an organic solvent, and drying and concentration of an organic layer. The isolated compound (18) can be further purified by chromatography, recrystallization and so on.

Some of intermediates used for the production of the compound of the present invention are commercially available or are compounds disclosed in known literatures and so on. Such intermediates can be produced, for example, by the following methods.

### (Reference Synthesis Process 1)

The compound (3) and the compound (4) can be produced by a method shown in the following scheme: 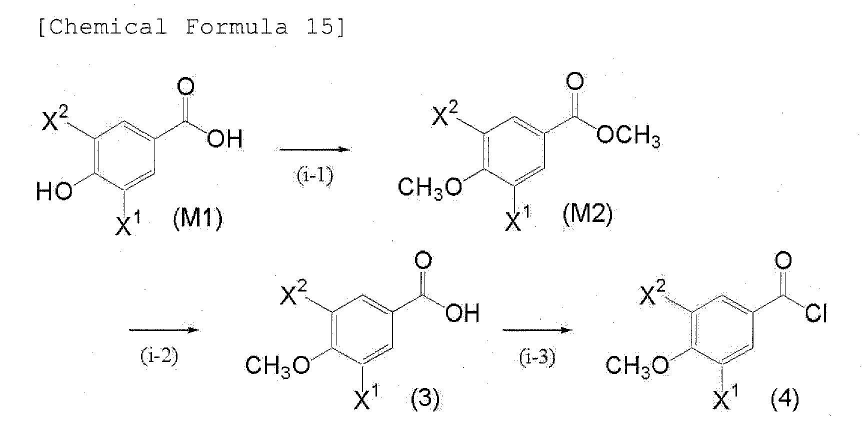 wherein X¹ and X² are as defined above.

### Step (i-1)

The compound (M2) can be produced by reacting the compound (M1) with a methylating agent such as methyl iodide or dimethyl sulfate in the presence of a base.

The reaction is usually carried out in the presence of a solvent.

Examples of the solvent used for the reaction include acid amides such as DMF; and sulfoxides such as DMSO.

Examples of the base used for the reaction include alkali metal carbonates such as sodium carbonate, potassium carbonate and cesium carbonate; and alkali metal hydroxides such as sodium hydroxide.

The used amount of the methylating agent is usually 2 to 5 mol per 1 mol of the compound (M1), and the used amount of the base is usually 2 to 5 mol per 1 mol of the compound (M1).

The reaction temperature is usually within a range from 0 to 140°C, and the reaction time is usually within a range from 0.5 to 24 hours.

After completion of the reaction, the compound (M2) can be isolated by post-treatment such as extraction of the reaction mixture with an organic solvent, and drying and concentration of an organic layer. The isolated compound (M2) can be further purified by chromatography, recrystallization and so on.

### Step (i-2)

The compound (3) can be produced by hydrolysis reaction of the compound (M2) in the presence of a base.

The reaction is usually carried out in the presence of a solvent.

Examples of the base used for the reaction include alkali metal hydroxides such as sodium hydroxide and potassium hydroxide.

Examples of the solvent used for the reaction include ethers such as THF, ethylene glycol dimethyl ether and tert-butyl methyl ether; alcohols such as methanol and ethanol; water; and their mixtures.

The used amount of the base is usually 1 to 10 mol per 1 mol of the compound (M2).

The reaction temperature is usually within a range from 0 to 120°C, and the reaction time is usually within a range from 0.5 to 24 hours.

After completion of the reaction, the reaction solution is made acidic. When a solid is precipitated, the compound (3) can be isolated by filtration. When a solid is not precipitated, the compound (3) can be isolated by post-treatment such as extraction of the reaction mixture with an organic solvent, and drying and concentration of an organic layer. The isolated compound (3) can be further purified by chromatography, recrystallization and so on.

### Step (i-3)

The compound (4) can be produced by reacting the compound (3) with a chlorinating agent such as thionyl chloride.

The reaction is usually carried out in the presence of a solvent.

Examples of the solvent used for the reaction include aliphatic hydrocarbons such as hexane, heptane and octane; aromatic hydrocarbons such as toluene and xylene; nitriles such as acetonitrile; halogenated hydrocarbons such as chlorobenzene; acid amides such as DMF; and tehir mixtures.

The used amount of a chlorinating agent such as thionyl chloride is usually 1 to 2 mol per 1 mol of the compound (3).

The reaction temperature is usually within a range from 20 to 120°C, and the reaction time is usually within a range from 0.1 to 24 hours.

After completion of the reaction, the compound (4) can be isolated by post-treatment such as concentration of the reaction mixture. The isolated compound (4) can be further purified by chromatography, recrystallization and so on.

### (Reference Synthesis Process 2)

The compound (10) can be produced by a method shown in the following scheme: 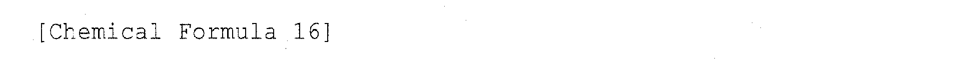 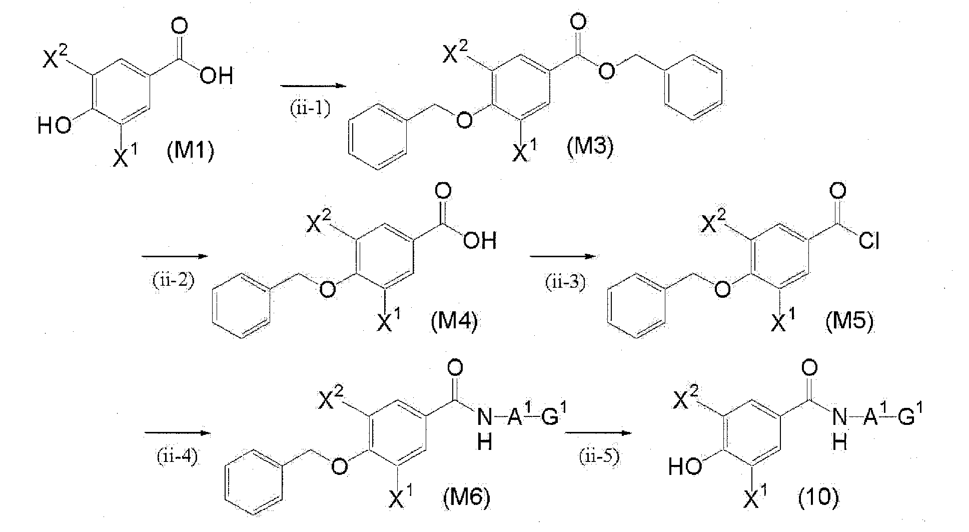 wherein A¹, X¹, X² and G¹ are as defined above.

### Step (ii-1)

The compound (M3) can be produced by reacting the compound (M1) with benzyl bromide in the presence of a base.

The reaction is usually carried out in the presence of a solvent.

Examples of the solvent used for the reaction include acid amides such as DMF; and sulfoxides such as DMSO.

Examples of the base used for the reaction include alkali metal carbonates such as sodium carbonate, potassium carbonate and cesium carbonate; and alkali metal hydroxides such as sodium hydroxide.

The used amount of benzyl bromide is usually 2 to 5 mol per 1 or the compound (M1), and the used amount of the base is usually 2 to 5 mol per 1 mol of the compound (M1).

The reaction temperature is usually within a range from 0 to 140°C, and the reaction time is usually within a range from 0.5 to 24 hours.

After completion of the reaction, the compound (M3) can be isolated by post-treatment such as extraction of the reaction mixture with an organic solvent, and drying and concentration of an organic layer. The isolated compound (M3) can be further purified by chromatography, recrystallization and so on.

### Step (ii-2)

The compound (M4) can be produced by hydrolysis reaction of the compound (M3) in the presence of a base.

The reaction is usually carried out in the presence of a solvent.

Examples of the base used for the reaction include alkali metal hydroxides such as sodium hydroxide and potassium hydroxide.

Examples of the solvent used for the reaction include ethers such as tetrahydrofuran, ethylene glycol dimethyl ether and MTBE; alcohols such as methanol and ethanol; water; and their mixtures.

The used amount of the base is usually 1 to 10 mol per 1 mol of the compound (M3).

The reaction temperature is usually within a range from 0 to 120°C, and the reaction time is usually within a range from 0.5 to 24 hours.

After completion of the reaction, the reaction solution is made acidic. when a solid is precipitated, the compound (M4) can be isolated by filtration. When a solid is not precipitated, the compound (M4) can be isolated by post-treatment such as extraction of the reaction mixture with an organic solvent, and drying and concentration of an organic layer. The isolated compound (M4) can be further purified by chromatography, recrystallization of the like.

### Step (ii-3)

The compound (M5) can be produced by reacting the compound (M4) with a chlorinating agent such as thionyl chloride.

The reaction is usually carried out in the presence of a solvent.

Examples of the solvent used for the reaction include aliphatic hydrocarbons such as hexane, heptane and octane; aromatic hydrocarbons such as toluene and xylene; nitriles such as acetonitrile; halogenated hydrocarbons such as chlorobenzene; acid amides such as DMF; and their mixtures.

The used amount of the chlorinating agent is usually 1 to 2 mol per 1 mol of the compound (M4).

The reaction temperature is usually within a range from 20 to 120°C, and the reaction time is usually within a range from 0.1 to 24 hours.

After completion of the reaction, the compound (M5) can be isolated by post-treatment such as concentration of the reaction mixture. The isolated compound (M5) can be further purified by chromatography, recrystallization and so on.

### Step (ii-4)

The compound (M6) can be produced by reacting the compound (M5) with the compound (2) in the presence of a base according to the method described in Synthesis Process 2.

### Step (ii-5)

The compound (10) can be produced by reacting the compound (M6) with hydrogen in the presence of palladium charcoal.

The reaction is usually carried out in the presence of a solvent.

Examples of the solvent used for the reaction include aliphatic hydrocarbons such as hexane, heptane and octane; aromatic hydrocarbons such as toluene and xylene; alcohols such as methanol and ethanol; esters such as ethyl acetate; ethers such as THF and MTBE; water; and their mixtures.

The used amount of palladium carbon is usually 0.01 to 0.1 mol per 1 mol of the compound (M6), and the used amount of hydrogen is usually 1 to 2 mol per 1 mol of the compound (M6).

The reaction temperature is usually within a range from 0 to 50°C, and the reaction time is usually within a range from 0.1 to 24 hours.

The pressure of hydrogen used in the reaction is in a range of normal pressure to 10 atoms.

After completion of the reaction, the compound (10) can be isolated by post-treatment such as filtration and concentration of the reaction mixture. The isolated compound (10) can be further purified by chromatography, recrystallization and so on.

Among the compounds (3), 3,5-difluoro-4-methoxybenzoic acid, which is the compound (3) wherein X¹ and X² are fluorine atoms, can be produced from 3,4,5-trifluorobenzaldehyde by a method described in Reference Synthesis Process 3 or Reference Synthesis Process 4.

### (Reference Synthesis Process 3)

3,5-Difluoro-4-methoxybenzoic acid can be produced by a method shown in the following scheme: 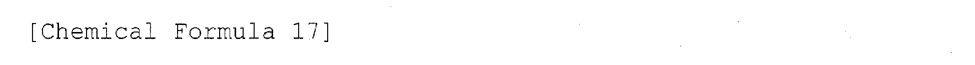 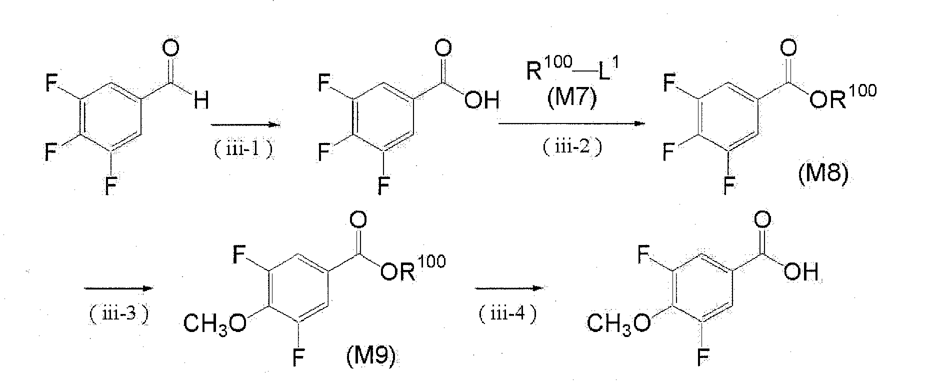 wherein R¹⁰⁰ represents a C1-C4 alkyl group, and L¹ represents a chlorine atom, a bromine atom, an iodine atom or a methanesulfonyloxy group.

### Step (iii-1)

3,4,5-Trifluorobenzoic acid can be produced by reacting 3,4,5-trifluorobenzaldehyde with an oxidizing agent.

The reaction is usually carried out in the presence of a solvent.

Examples of the solvent used for the reaction include ketones such as acetone, methyl ethyl ketone and methyl isobutyl ketone; nitriles such as acetonitrile; acid amides such as DMF; halogenated hydrocarbons such as chloroform; water; and their mixtures.

Examples of the oxidizing agent used for the reaction include potassium permanganate, 3-chloroperbenzoic acid, and a peroxo monosulfuric acid compound [OXONE (registed trademark)].

The used amount of the oxidizing agent is usually 1 to 5 mol per 1 mol of 3,4,5-trifluorobenzaldehyde.

The reaction temperature is usually within a range from 0 to 100°C, and the reaction time is usually within a range from 0.5 to 24 hours.

After completion of the reaction, 3,4,5-trifluorobenzoic acid can be isolated by post-treatment such as extraction of the reaction mixture with an organic solvent, and drying and concentration of an organic layer. The isolated 3,4,5-trifluorobenzoic acid can be further purified by chromatography, recrystallization, and so on.

### Step (iii-2)

The compound (M8) can be produced by reacting 3,4,5-trifluorobenzoic acid with the compound (M7) in the presence of a base.

The reaction is usually carried out in the presence of a solvent.

Examples of the solvent used for the reaction include nitriles such as acetonitrile; acid amides such as DMF; sulfoxides such as DMSO; ketones such as acetone, methyl ethyl ketone and methyl isobutyl ketone; water; and their mixtures.

Examples of the base used for the reaction include alkali metal carbonates such as sodium carbonate, potassium carbonate and cesium carbonate; and alkali metal hydroxides such as sodium hydroxide and potassium hydroxide.

The used amount of the compound (M7) is usually 2 to 5 mol per 1 mol of 3,4,5-trifluorobenzoic acid, and the used amount of the base is usually 2 to 5 mol per 1 mol of 3,4,5-trifluorobenzoic acid.

The reaction temperature is usually within a range from 0 to 140°C, and the reaction time is usually within a range from 0.5 to 24 hours.

After completion of the reaction, the compound (M8) can be isolated by post-treatment such as extraction of the reaction mixture with an organic solvent, and drying and concentration of an organic layer. The isolated compound (M8) can be further purified by chromatography, recrystallization, and so on.

### Step (iii-3)

The compound (M9) can be produced by reacting the compound (M8) with methanol in the presence of a base.

The reaction is usually carried out in the presence of a solvent.

Examples of the solvent used for the reaction include nitriles such as acetonitrile; acid amides such as DMF; sulfoxides such as DMSO; ketones such as acetone, methyl ethyl ketone and methyl isobutyl ketone; hydrocarbons such as hexane and toluene; methanol; and tehir mixtures.

Examples of the base used for the reaction include alkali metal carbonates such as sodium carbonate, potassium carbonate and cesium carbonate; alkali metal hydroxides such as sodium hydroxide; and alkali metal hydrides such as sodium hydride.

The used amount of methanol is usually 1 to 10 mol per 1 mol of the compound (M8), and the used amount of the base is usually 1 to 5 mol per 1 mol of the compound (M8).

The reaction temperature is usually within a range from 0 to 140°C, and the reaction time is usually within a range from 0.5 to 24 hours.

After completion of the reaction, the compound (M9) can be isolated by post-treatment such as extraction of the reaction mixture with an organic solvent, and drying and concentration of an organic layer. The isolated compound (M9) can be further purified by chromatography, recrystallization, and so on.

### Step (iii-4)

3,5-Difluoro-4-methoxybenzoic acid can be produced by hydrolysis reaction of the compound (M9) in the presence of a base.

The reaction is usually carried out in the presence of a solvent.

Examples of the base used for the reaction include alkali metal hydroxides such as sodium hydroxide and potassium hydroxide.

Examples of the solvent used for the reaction include ethers such as THF, ethylene glycol dimethyl ether and tert-butyl methyl ether; alcohols such as methanol and ethanol; water; and their mixtures.

The used amount of the base is usually 1 to 10 mol per 1 mol of the compound (M9).

The reaction temperature is usually within a range from 0 to 120°C, and the reaction time is usually within a range from 0.5 to 24 hours.

After completion of the reaction, the reaction solution is made acidic. When a solid is precipitated, 3,5-difluoro-4-methoxybenzoic acid can be isolated by filtration. When a solid is not precipitated, 3,5-difluoro-4-methoxybenzoic acid can be isolated by post-treatment such as extraction of the reaction mixture with an organic solvent, and drying and concentration of an organic layer.

The isolated 3,5-difluoro-4-methoxybenzoic acid can be further purified by chromatography, recrystallization, and so on.

### (Reference Synthesis Process 4)

3,5-Difluoro-4-methoxybenzoic acid can be produced by a method shown in the following scheme.

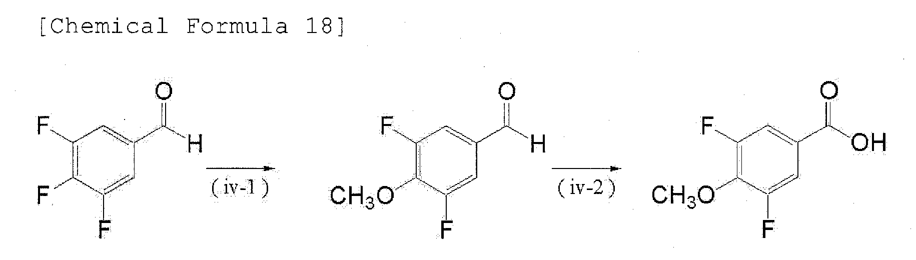

### Step (iv-1)

3,5-Difluoro-4-methoxybenzaldehyde can be produced by reacting 3,4,5-trifluorobenzaldehyde with methanol in the presence of a base.

The reaction is usually carried out in the presence of a solvent.

Examples of the solvent used for the reaction include hydrocarbons such as toluene; nitriles such as acetonitrile; acid amides such as DMF; sulfoxides such as DMSO; ketones such as acetone, methyl ethyl ketone and methyl isobutyl ketone; water; and their mixtures.

Examples of the base used for the reaction include alkali metal carbonates such as sodium carbonate, potassium carbonate and cesium carbonate; alkali metal hydroxides such as sodium hydroxide; and alkali metal hydrides such as sodium hydride.

The used amount of methanol is usually 1 to 10 mol per 1 mol of 3,4,5-trifluorobenzaldehyde, and the used amount of the base is usually 1 to 5 mol per 1 mol of 3,4,5-trifluorobenzaldehyde.

The reaction temperature is usually within a range from 0 to 100°C, and the reaction time is usually within a range from 0.5 to 24 hours.

After completion of the reaction, 3,5-difluoro-4-methoxybenzaldehyde can be isolated by post-treatment such as extraction of the reaction mixture with an organic solvent, and drying and concentration of an organic layer. The isolated 3,5-difluoro-4-methoxybenzaldehyde can be further purified by chromatography, recrystallization, and so on.

### Step (iv-2)

3,5-Difluoro-4-methoxybenzoic acid can be produced by reacting 3,5-difluoro-4-methoxybenzaldehyde with an oxidizing agent.

The reaction is usually carried out in the presence of a solvent.

Examples of the solvent used for the reaction include ketones such as acetone, methyl ethyl ketone and isobutyl ketone; nitriles such as acetonitrile; acid amides such as DMF; halogenated hydrocarbons such as chloroform; water; and their mixtures.

Examples of the oxidizing agent used for the reaction include potassium permanganate, 3-chloroperbenzoic acid, and a peroxo monosulfulic acid compound [OXONE(registered trademark)].

The used amount of the oxidizing agent is usually 1 to 3 mol per 1 mol of 3,5-difluoro-4-methoxybenzaldehyde.

The reaction temperature is usually within a range from 0 to 100°C, and the reaction time is usually within a range from 0.5 to 24 hours.

After completion of the reaction, 3,5-difluoro-4-methoxybenzoic acid can be isolated by post-treatment such as extraction of the reaction mixture with an organic solvent, and drying and concentration of an organic layer. The isolated 3,5-difluoro-4-methoxybenzoic acid can be further purified by chromatography, recrystallization, and so on.

The plant disease controlling agent of the present invention is usually in the form of formulations such as an emulsifiable concentrate, a wettable powder, a granular wettable powder, a flowable formulation, a dust or a granule produced by mixing an amide compound represented by the above formula (1) with an inert carrier such as a solid carrier or a liquid carrier and, if necessary, a surfactant and other auxiliary agents for formulation.

The plant disease control agent may contain, in addition to an amide compound represented by the above formula (1), other control agents such as fungicides, insecticides, acaricides and nematocides, and additives such as herbicides, plant growth regulators, fertilizers or soil conditioners. Specific examples of other control agents and additives are described later.

The plant disease control agent usually contains an amide compound represented by the above formula (1) in an amount of 0.1% to 90% by weight.

Examples of the solid carrier used in formulation include fine powders or particles of minerals such as kaolin clay, attapulgite clay, bentonite, montmorillonite, acid clay, pyrophyllite, talc, diatomaceous earth, and calcite; natural organic substances such as corncob powder, and walnut shell flour; synthetic organic substances such as urea; salts such as calcium carbonate, and ammonium sulfate; and synthetic inorganic substances such as synthetic hydrated silicon oxide.

Examples of the liquid carrier used in formulation include aromatic hydrocarbons such as xylene, alkylbenzene and methylnaphthalene; alcohols such as 2-propanol, ethylene glycol, propylene glycol and cellosolve; ketones such as acetone, cyclohexanone and isophorone; vegetable oils such as soybean oil and cotton seed oil; petroleum aliphatic hydrocarbons; esters; and dimethyl sulfoxide, acetonitrile and water.

Examples of the surfactant used in formulation include anionic surfactants such as alkyl sulfate, alkyl aryl sulfonate, dialkyl sulfosuccinate, polyoxyethylene alkyl aryl ether phosphate, ligninsulfonate and a naphthalenesulfonate formaldehyde polycondensate; and nonionic surfactants such as polyoxyethylene alkyl aryl ether, polyoxyethylene alkyl polyoxypropylene block copolymers and sorbitan fatty acid esters.

Examples of the auxiliary agent for formulation include water-soluble polymers such as polyvinyl alcohol and polyvinyl pyrrolidone; polysaccharides such as gum Arabic, alginic acid and a salt thereof, CMC(carboxymethyl cellulose) and xanthan gum; inorganic substances such as aluminum magnesium silicate, and alumina sol; preservatives; colorants; PAP (acidic isopropyl phosphate); and stabilizers such as BHT.

The plant disease controlling agent of the present invention can be used as a controlling agent for plant diseases in crop lands such as upland fields, paddy fields, lawn, and orchards, etc. The plant disease controlling agent can control plant diseases in crop lands where the following "crops" or the like are cultivated.

Field crops: corn, rice, wheat, barley, rye, oat, sorghum, cotton, soybean, peanut, buckwheat, beet, rape, sunflower, sugarcane, tobacco, etc.;
Vegetables: solanaceae (e.g. eggplant, tomato, green pepper, pepper and potato), Cucurbitaceae (e.g. cucumber, pumpkin, zucchini, watermelon and melon), Cruciferae (e.g. Japanese radish, turnip, horseradish, kohlrabi, Chinese cabbage, cabbage, leaf mustard, broccoli and cauliflower), Compositae (e.g. edible burdock, garland chrysanthemum, globe artichoke and lettuce), Liliacede (e.g., Welsh onion, onion, garlic and asparagus), Umbelliferae (e.g. carrot, parsley, celery and pastinaca), Chenopodiaceae (e.g. spinach and chard), Lamiaceae (e.g. perilla, mint and basil), strawberry, sweet potato, Chinese yam, taro, etc.;
Flowers and ornament plants;
Ornamental foliage plants;
Fruit trees: pomaceous fruits (e.g. apple, pear, Japanese pear, Chinese quince and quince), stone fruits (e.g. peach, plum, nectarine, Japanese apricot, cherry, apricot and prune), citrus fruits (e.g. satsuma mandarin, orange, lemon, lime and grapefruit), nut trees (e.g. chestnut, walnut, hazel, almond, pistachio, cashew nut and macadamia nut), berries (blueberry, cranberry, blackberry and raspberry), grape, Japanese persimmon, olive, loquat, banana, coffee, date palm, coconut palm, etc.;
Trees other than fruit trees: tea, mulberry, flowering trees and shrubs, street trees (e.g. Japanese ash, birch, flowering dogwood, blue gum, ginkgo, lilac, maple, oak, poplar, Chinese redbud, Formosa sweet gum, plane tree, zelkova, Japanese arborvitae, fir, Japanese hemlock, needle juniper, pine, Japanese spruce and Japanese yew), etc.

The above-described "crops" include crops having resistance to herbicides such as HPPD inhibitors (e.g. isoxaflutole), ALS inhibitors (e.g. imazethapyr and thifensulfuron-methyl), EPSP synthetase inhibitors, glutamine synthetase inhibitors, and bromoxynil which has been imparted by a classic breeding method or a genetic recombination technology.

Examples of the "crops" having the resistance imparted by a classic breeding method include Clearfield® canola resistant to imidazolinone herbicides (e.g. imazethapyr) and STS soybean resistant to sulfonylurea ALS inhibition type herbicides (e.g. thifensulfuron-methyl). Examples of the "crops" having the resistance imparted by a genetic recombination technology include corn cultivars resistant to glyphosate and glufosinate, which are already on the market under the trade names of RoundupReady® and LibertyLink®.

The above-described "crops" include crops which have been enabled by a genetic recombination technology to synthesize a selective toxin known in the case of, for example, *Bacillus*.

Examples of toxins produced in such genetically modified plants include insecticidal proteins derived from *Bacillus cereus* and *Bacillus popilliae;* insecticidal proteins such as δ-endotoxins (e.g. Cry1Ab, Cry1Ac, Cry1F, Cry1Fa2, Cry2Ab, Cry3A, Cry3Bb1 and Cry9C), VIP 1, VIP 2, VIP 3, VIP 3A, etc., which are derived from *Bacillus thuringiensis;* toxins derived from nematodes; toxins produced by animals, such as scorpion toxin, spider toxin, bee toxin, insect-specific neurotoxins, etc.; filamentous fungi toxins; plant lectins; agglutinin; protease inhibitors such as trypsin inhibitors, serine protease inhibitors, patatin, cystatin, papain inhibitors, etc.; ribosome-inactivating proteins (RIPs) such as ricin, corn-RIP, abrin, rufin, sapolin, briodin, etc.; steroid metabolic enzymes such as 3-hydroxysteroid oxidase, ecdysteroid-UDP-glucosyltransferase, cholesterol oxidase, etc.; ecdysone inhibitors; HMG-COA reductase; ion channel inhibitors such as sodium channel inhibitors, calcium channel inhibitors, etc.; juvenile hormone esterase; diuretic hormone receptors; stilbene synthetase; bibenzyl synthetase; chitinase; and glucanase.

The toxins produced in such genetically modified crops also include hybrid toxins, partly deficient toxins and modified toxins of the above-described insecticidal proteins

The hybrid toxins are produced by a novel combination of different domains of such proteins.

As the partly deficient toxin, Cry1Ab deficient in a part of the amino acid sequence is known. In the modified toxins, one or more amino acids of a natural toxin have been replaced.

Examples of such toxins and genetically modified plants capable of synthesizing such toxins are described in EP-A-0 374 753, WO 93/07278, WO 95/34656, EP-A-0 427 529, EP-A-451 878, WO 03/052073, etc.

The toxins contained in such genetically modified plants impart resistance to insect pests of, in particular, Coleoptera, Diptera and Lepidoptera to the plants.

Genetically modified plants containing one or more insecticidal insect-resistant genes and capable of producing one or more toxins have already been known, and some of them are on the market. Examples of such genetically modified plants include YieldGard® (a corn cultivar capable of producing Cry1Ab toxin), YieldGard Rootworm® (a corn cultivar capable of producing Cry3Bb1 toxin), YieldGard Plus® (a corn cultivar capable of producing Cry1Ab and Cry3Bb1 toxins), Herculex I® (a corn cultivar capable of producing phosphinotrysin N-acetyltransferase (PAT) for imparting resistance to Cry1Fa2 toxin and Glyfosinate), NuCOTN33B (a cotton cultivar capable of producing Cry1Ac toxin), Bollgard I® (a cotton cultivar capable of producing Cry1Ac toxin), Bollgard II® (a cotton cultivar capable of producing Cry1Ac and Cry2Ab toxins), VIPCOT® (a cotton cultivar capable of producing VIP toxin), NewLeaf® (a potato cultivar capable of producing Cry3A toxin), NatureGard®, Agrisure®, GT Advantage (GA21 glyphosate resistant properties), Agrisure® CB Advantage (Bt11 corn borer (CB) properties), and Protecta®.

The above-described "crops" also include crops having ability to produce an anti-pathogenic substance having a selective action which has been imparted by a genetic recombination technology.

As examples of the anti-pathogenic substance, PR proteins (PRPs) and the like are known. Such anti-pathogenic substances and genetically modified plants capable of producing them are described in EP-A-0 392 225, WO 95/33818, EP-A-0 353 191, etc.

Examples of such anti-pathogenic substances produced in the genetically modified plants include ion channel inhibitors such as sodium channel inhibitors, calcium channel inhibitors (for example, KP1, KP4, and KP6 toxins produced by viruses are known), etc.; stilbene synthase; bibenzyl synthase; chitinase; glucanase; PR proteins; and anti-pathogenic substances produced by microorganisms, such as peptide antibiotics, antibiotics having a heterocyclic ring, protein factors concerned in resistance to plant diseases (which are called plant disease-resistant genes and are described in WO 03/000906), etc.

Plant diseases against which the plant disease control agent of the present invention exerts an excellent effect include plant diseases caused by fungi, bacteria and viruses. Specific examples of the fungi include *Erysiphe* such as wheat powdery mildew (*Erysiphe graminis*), *Uncinula spp.* such as grape powdery mildew (*Uncinula necator*), *Podosphaera spp*. such as apple powdery mildew (*Podosphaera leucotricha*), *Sphaerotheca spp*. such as cucumber powdery mildew (*Sphaerotheca cucurbitae*), *Oidiopsis spp*. such as tomato powdery mildew (*Oidiopsis sicula*), *Magnaporthe spp.* such as rice blast (*Magnaporthe oryzae*), *Cochliobolus spp.* such as rice brown spot (*Cochliobolus miyabeanus*), *Mycosphaerella spp.* such as wheat leaf blotch (*Mycosphaerella graminicola*), *Pyrenophora spp.* such as barley net blotch (*Pyrenophora teres*), *Stagonospora spp.* such as wheat Glume blotch (*Stagonospora nodorum*), *Rhynchosporium spp.* such as barley scald (*Rhynchosporium secalis*), *Pseudocercosporella spp*. such as wheat eyespot (*Pseudocercosporella herpotrichoides*), Gaeumannomyces *spp.* such as wheat take-all (*Gaeumannomyces graminis*), *Fusarium* such *spp*. as wheat Fusarium head blight (*Fusarium* spp.), *Microdochium spp.* such as wheat snow mold (*Microdochium nivale*), *Venturia spp*. such as apple scab (*Venturia inaequalis*), *Elsinoe spp*. such as grape anthracnose (*Elsinoe ampelina*), *Botrytis spp*. such as cucumber gray mold (*Botrytis cinerea*), *Monilinia spp*. such as peach brown rot (*Monilinia fructicola*), *Phoma spp*. such as rape stem canker (*Phoma lingam*), *Cladosporium spp*. such as tomato leaf mold (*Cladosporium fulvum*), *Cercospora spp*. such as sugarbeet brown spot (*Cercospora beticola*), *Cercosporidium spp*. such as peanut late leaf spot (*Cercosporidium personatum*), *Colletotrichum spp*. such as strawberry anthracnose (*Colletotrichum fragariae*), *Sclerotinia spp*. such as cucumber stem rot (*Sclerotinia sclerotiorum*), *Alternaria spp*. such as apple necrotic leaf spot (*Alternaria mali*), *Verticillium spp.* such as eggplant verticillium wilt (*Verticillium dahliae*), *Rhizoctonia spp.* such as rice sheath blight (*Rhizoctonia solani*), *Puccinia spp*. such as wheat leaf rust (*Puccinia recondita*), *Phakopsora spp.* such as soybean rust (*Phakopsora pachyrhizi*), *Tilletia spp.* such as wheat bunt (*Tilletia caries*), *Ustilago spp.* such as barley loose smut (*Ustilago nuda*), *Sclerotium spp.* such as peanut southern blight (*Sclerotium rolfsii*), *Phytophthora spp.* such as potato late blight (*Phytophthora infestans*), *Pseudoperonospora spp.* such as cucumber downy mildew (*Pseudoperonospora cubensis*), *Peronospora spp.* such as Chinese cabbage downy mildew (*Peronospora parasitica*), *Plasmopara spp.* such as grape downy mildew (*Plamospara viticola), Sclerophthora spp.* such as rice downy mildew (*Sclerophthora macrospora*), *Pythium spp*. such as cucumber seedling damping-off (*Pythium ultimum*), and *Plasmodiophora spp.* such as rapeseed clubroot (*Plasmodiophora brassicae*).

Examples of the bacteria include Burkholderia such as bacterial rice seedling blight (*Burkholderia plantarii*), *Pseudomonas* such as bacterial cucumber leaf spot (*Pseudomonas syringae pv. Lachrymans*), *Ralstonia* such as eggplant wilting (*Ralstonia solanacearum*), *Xanthomonas* such as Asiatic citrus canker (*Xanthomonas citiri*), and *Erwinia* such as Chinese cabbage bacterial soft rot (*Erwinia carotovora*).

Examples of the viruses include Tobacco mosaic virus and Cucumber mosaic virus. However, the sterilizing spectra should not be limited thereto in any cases.

The plant disease controlling agent of the present invention or an amide compound represented by the formula (1) can be used in foliage treatment to protect plants from plant diseases, or can be also used for in soil treatment to protect plants growing in the soil from plant diseases.

A plant disease control method which comprises applying an effective amount of an amide compound represented by the formula (1) to plants or soil, and use of an amide compound represented by the formula (1) for control of plant diseases by foliage treatment or applying to foliage treatment are included in the present invention.

In the plant disease control method of the present invention and the use of the present invention, aspects and preferred kinds of an amide compound represented by the formula (1) are the same as those contained in the plant disease control agent of the present invention.

In the plant disease control method, the plant disease control agent of the present invention may be used as the amide compound represented by the formula (1).

The plant disease controlling method and the use of the present invention can be carried out by treating the foliage of a plant in which onset of diseases is presumed or the soil where the plant is growing, or the foliage of a plant in which onset of diseases has been confirmed or the soil where the plant is growing with an effective amount of an amide compound represented by the formula (1).

In the plant disease control agent and the use of the present invention, the effective amount of an amide compound represented by the formula (1) varies depending upon the kind of crops as plants to be protected, the kind of diseases to be controlled, severity of diseases, form of the formulation, time of application, weather conditions and the like. In the case of soil treatment, the effective amount of an amide compound represented by the formula (1) is usually within a range of from 1 to 5,000 g, and preferably 5 to 1,000 g per 10,000 m² of soil.

In the plant disease control agent and the use of the present invention, when an amide compound represented by the formula (1) is formulated into a liquid formulation such as an emulsifiable concentrate, a wettable powder or a flowable formulation, the formulation is usually used by spraying after dilution with water.

In this case, the concentration of the amide compound in a spray solution is usually within a range of from 0.0001 to 3% by weight, and preferably from 0.0005 to 1% by weight.

When an amide compound represented by the formula (1) is formulated into a solid formulation such as a dust or a granule, the formulation is usually used for a treatment without being diluted.

The plant disease control agent and the use of the present invention include use of an amide compound represented by the formula (1) for seed disinfection.

Examples of a method for seed disinfection include a method of immersing seeds of plants in a liquid formulation containing the amide compound, a method of spraying or smearing a liquid formulation containing the amide compound over seeds of plants, and a method of dust coating of seeds of plants using a solid formulation containing the amide compound.

In the liquid formulation, the concentration of the amide compound is preferably from 1 to 1,000 ppm.

In the plant disease control method and the use of the present invention, an amide compound represented by the above formula (1) can be used in combination with other control agents such as fungicides, insecticides, acaricides and nematocides, and additives such as herbicides, plant growth regulators, fertilizers or soil conditioners. The plant disease control agent of the present invention can also be used in combination with fungicides, insecticides, acaricides and nematocides, and additives such as herbicides, plant growth regulators, fertilizers and soil conditioners, or can be used simultaneously without mixing with the other control agents or the additives.

Examples of the active ingredients of other control agents include chlorothalonil, fluazinam, dichlofluanid, fosetyl-Al, cyclic imide derivatives (e.g., captan, captafol, folpet, etc.), dithiocarbamate derivatives (e.g., maneb, mancozeb, thiuram, ziram, zineb, propineb, etc.), inorganic or organic copper derivatives (e.g., basic copper sulfate, basic copper chloride, copper hydroxide, oxine-copper, etc.), acylalanine derivatives (e.g., metalaxyl, furalaxyl, ofurace, cyprofuram, benalaxyl, oxadixyl, etc.), strobilurine like compound (e.g., kresoxim-methyl, azoxystrobin, trifloxystrobin, picoxystrobin, pyraclostrobin, fluoxastrobin, metominostrobin, orysastrobin, enestrobin, dimoxystrobin, etc.), anilinopyrimidine derivatives (e.g., cyprodinil, pyrimethanil, mepanipyrim, etc.), phenyl pyrrole derivatives (e.g., fenpiclonil, fludioxonil, etc.), imide derivatives (e.g., procymidone, iprodione, vinclozolin, etc.), benzimidazole derivatives (e.g., carbendazim, benomyl, thiabendazole, thiophanate methyl, etc.), amine derivatives (e.g., fenpropimorph, tridemorph, fenpropidin, spiroxamine, etc.), azole derivatives (e.g., propiconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, flutriafol, ipconazole, pefurazoate, prothioconazole, etc.), triforine, pyrifenox, fenarimol, propamocarb, cymoxanil, dimethomorph, flumorph, famoxadone, fenamidone, pyribencarb, iprovalicarb, benthiavalicarb, mandipropamid, cyazofamid, amisulbrom, zoxamide, ethaboxam, boscalid, penthiopyrad, fluopyram, bixafen, carboxin, oxycarboxin, thifluzamide, flutolanil, mepronil, furametpyr, pencycuron, hymexazol, etridiazole, ferimzone, silthiofam, blasticidin S, kasugamycin, streptomycin, pyrazophos, iprobenfos, edifenphos, isoprothiolane, fthalide, pyroquilon, tricyclazole, carpropamid, diclocymet, fenoxanil, probenazole, tiadinil, isotianil, iminoctadine, guazatine, tolnifanide, tolclophos-methyl, fenhexamid, polyoxin B, quinoxyfen, proquinazid, metrafenone, cyflufenamid, diethofencarb, fluopicolide and acibenzolar-S-methyl.

### Examples

The present invention will be explained in more detail by way of Formulation Examples, Test Example and so on, which the present invention is not limited to.

First, Synthesis Examples of the compound of the present invention are shown.

### Synthesis Example 1

To 5 ml of DMF were added 0.45 g of 3,4-dimethoxybenzoic acid, 0.50 g of WSC and 0.30 g of cyclohexylmethylamine, followed by stirring at 100°C for 2 hours. To the reaction mixture cooled to about room temperature, dilute hydrochloric acid was added, followed by extraction with ethyl acetate. The organic layer was washed with an aqueous sodium hydrogen carbonate solution, dried over magnesium sulfate and then concentrated under reduced pressure. The residue was subjected to silica gel column chromatography, and 0.19 g of N-(cyclohexylmethyl)-3,4-dimethoxybenzamide was obtained (hereinafter, referred to as the present compound 1).

### Present compound 1

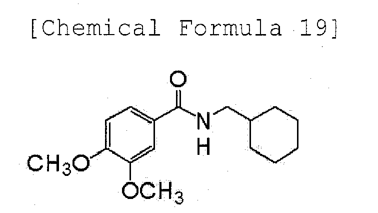

¹H-NMR(CDCl₃) δ: 0.95-1.29(5H, m), 1.55-1.79 (6H, m), 3.29(2H, dd, J=6.5, 6.5Hz), 3.91(3H, s), 3.92(3H, s), 6.21(1H, br s), 6.85(1H, d, J=8.5Hz), 7.26(1H, dd, J=8.5, 2.0Hz), 7.44(1H, d, J=2.0Hz).

### Synthesis Example 2

In the same manner as in Synthesis Example 1, using 3,4,5-trimethoxybenzoic acid in place of 3,4-dimethoxybenzoic acid, N-(cyclohexylmethyl)-3,4,5-trimethoxybenzamide (hereinafter, referred to as the present compound 2) was obtained.

### Present compound 2

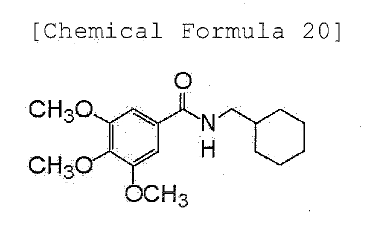

¹H-NMR(CDCl₃) δ: 0.95-1.31 (5H, m), 1.55-1.79(6H, m), 3.29 (2H, dd, J = 6.5, 6.5 Hz), 3.88 (3H, s), 3.91 (6H, s), 6.13 (1H, br s), 6.99 (2H, s).

### Synthesis Example 3

To 5 ml of THF were added 0.30 g of 3,4-dimethoxybenzoyl chloride, 0.21 g of (1S)-1-cyclohexylethylamine and 0.23 g of triethylamine, followed by stirring at room temperature for 4 hours. To the reaction mixture, ethyl acetate was added and insolubles were filtered off through Celite. The filtrate was concentrated under reduced pressure. The residue was washed with MTBE,and N-((1S)-1-cyclohexylethyl)-3,4-dimethoxybenzamide was obtained (hereinafter, referred to as the present compound 3).

### Present compound 3

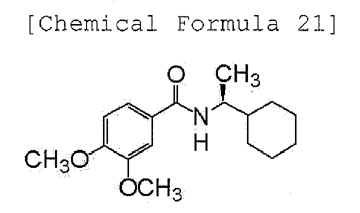

¹H-NMR (CDCl₃) δ: 0.99-1.49 (8H, m), 1.54-1.87 (6H, m), 3.92 (3H, s), 3.94 (3H, s), 4.01-4.13 (1H, m), 5.82-5.90 (1H, m), 6.85 (1H, d, J = 8.4 Hz), 7.23 (1H, dd,J=8.4, 2.0 Hz), 7.44 (1H, d, J = 2.0 Hz).

### Synthesis Example 4

In the same manner as in Synthesis Example 3, using 3,4,5-trimethoxybenzoyl chloride in place of 3,4-dimethoxybenzoyl chloride, N-((1S)-1-cyclohexylethyl)-3,4,5-trimethoxybenzamide (hereinafter referred to as the present compound 4) was obtained.

### Present compound 4

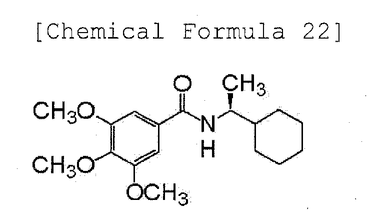

¹H-NMR (CDCl₃) δ: 0.97-1.50 (8H, m), 1.64-1.85 (6H, m), 3.88 (3H, s), 3.91 (6H, s), 4.02-4.11 (1H, m), 5.83-5.85 (1H, br m), 6.98 (2H, s).

### Synthesis Example 5

In the same manner as in Synthesis Example 3, using cyclohexylamine in place of (1S)-1-cyclohexylethylamine and using 3,4,5-trimethoxybenzoic acid in place of 3,4-dimethoxybenzoic acid, N-(cyclohexyl)-3,4,5-trimethoxybenzamide (hereinafter, referred to as the present compound 5) was obtained.

### Present compound 5

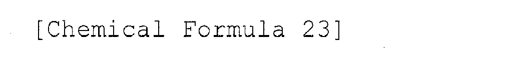

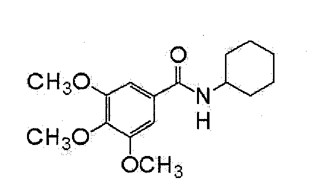

¹H-NMR (CDCl₃) δ: 1.15-1.30 (3H, m), 1.38-1.50 (2H, m), 1.63-1.81 (3H, m), 2.00-2.08 (2H, m), 3.87 (3H, s), 3.89-4.01 (1H, m), 3.91 (6H, s), 5.82-5.89 (1H, br m), 6.96 (2H, s).

### Synthesis Example 6

In the same manner as in Synthesis Example 3, using cyclohexylamine in place of (1S)-1-cyclohexylethylamine, N-(cyclohexyl)-3,4-dimethoxybenzamide (hereinafter, referred to as the present compound 6) was obtained.

### Present compound 6

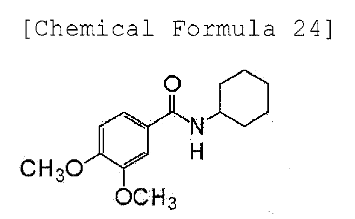

¹H-NMR (CDCl₃) δ: 1.15-1.29 (3H, m), 1.37-1.50 (2H, m), 1.62-1.80 (3H, m), 1.99-2.08 (2H, m), 3.91-4.01 (1H, m), 3.91 (3H, s), 3.93 (3H, s), 5.87-5.93 (1H, br m), 6.85 (1H, d, J = 8.4 Hz), 7.23 (1H, dd, J = 8.4, 2.0 Hz), 7.41 (1H, d, J = 2.0 Hz).

### Synthesis Example 7

To 5 ml of THF were added 0.40 g of 3,4-difluorobenzoyl chloride, 0.22 g of cyclohexylmethylamine and 0.40 g of triethylamine, followed by stirring at room temperature for 2 hours. To the reaction mixture, ethyl acetate was added and insolubles were filtered off through Celite. The filtrate was concentrated under reduced pressure. The residue was washed with MTBE, and 0.46 g of N-(cyclohexylmethyl)-3,4-difluorobenzamide was obtained. N-(cyclohexylmethyl)-3,4-difluorobenzamide 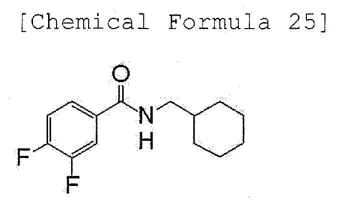 ¹H-NMR (CDCl₃) δ: 0.93-1.32 (5H, m), 1.52-1.80 (6H, m), 3.28 (2H, dd, J = 6.5, 6.5 Hz), 6.17 (1H, br s), 7.17-7.24 (1H, m), 7.48-7.52 (1H, m), 7.63 (1H, ddd, J = 10.7, 7.6, 2.3 Hz).

To 3 ml of methanol were added 0.30 g of N-(cyclohexylmethyl)-3,4-difluorobenzamide and 3 ml of a 28% solution of sodium methoxide in methanol. The mixed solution was subjected to reaction in a microwave reaction apparatus (manufactured by CEM Co. under the trade name of Discover) under 18 kgf/cm² at 100°C for 5 minutes. After cooling to about room temperature, the reaction mixture was poured into water, followed by extraction with ethyl acetate. The organic layer was dried over magnesium sulfate and concentrated under reduced pressure. The residue was subjected to silica gel column chromatography, and 63 mg of N-(cyclohexylmethyl)-3-fluoro-4-methoxybenzamide was obtained (hereinafter, referred to as the present compound 7).

### Present compound 7

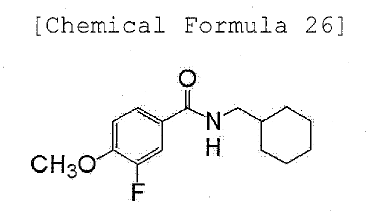

¹H-NMR (CDCl₃) δ: 0.92-1.32 (5H, m), 1.51-1.82 (6H, m), 3.28 (2H, dd, J = 6.4, 6.4 Hz), 3.93 (3H, s), 6.08 (1H, br s), 6.97 (1H, t, J = 8.2 Hz), 7.49-7.55 (2H, m).

### Synthesis Example 8

In the same manner as in Synthesis Example 7, using 2-methylcyclohexylamine in place of cyclohexylmethylamine and using 3,4,5-trifluorobenzoyl chloride in place of 3,4-difluorobenzoyl chloride, N-(2-methylcyclohexyl)-3,5-difluoro-4-methoxybenzamide (hereinafter, referred to as the present compound 8) was obtained.

### Present compound 8

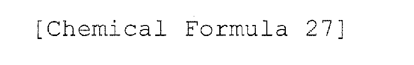

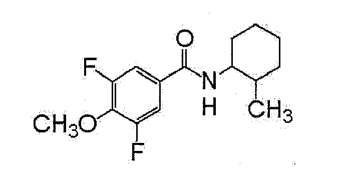

¹H-NMR (CDCl₃) δ: 0.93 (0.9H, d, J = 7.0 Hz), 0.97 (2.1H, d, J = 6.5 Hz), 1.12-2.05 (9.0H, m), 3.62-3.71 (0.7H, m), 4.05-4.06 (3.0H, m), 4.20-4.26 (0.3H, m), 5.70 (0.7H, d, J = 8.7 Hz), 5.96 (0.3H, d, J = 8.7 Hz), 7.28-7.35 (2.0H, m).

### Synthesis Example 9

In the same manner as in Synthesis Example 7, using 3,4,5-trifluorobenzoyl chloride in place of 3,4-difluorobenzoyl chloride, N-(cyclohexylmethyl)-3,5-difluoro-4-methoxybenzamide (hereinafter, referred to as the present compound 9) was obtained.

### Present compound 9

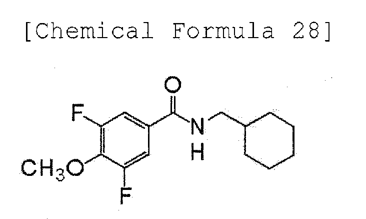

¹H-NMR (CDCl₃) δ: 0.94-1.04 (2H, m), 1.14-1.30 (3H, m), 1.54-1.79 (6H, m), 3.28 (2H, t, J = 6.4 Hz), 4.06-4.06 (3H, m), 6.01 (1H, br s), 7.28-7.35 (2H, m).

### Synthesis Example 10

In the same manner as in Synthesis Example 3, cyclohexylamine in place of (1S)-1-cyclohexylethylamine and using 3,5-difluoro-4-methoxybenzoyl chloride in place of 3,4-dimethoxybenzoyl chloride, N-(cyclohexyl)-3,5-difluoro-4-methoxybenzamide (hereinafter, referred to as the present compound 10) was obtained.

### Present compound 10

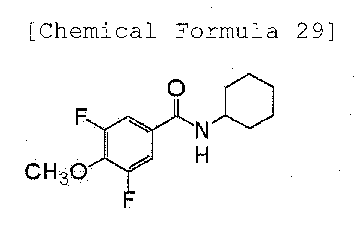

¹H-NMR (CDCl₃) δ: 1.16-1.27 (3H, m), 1.37-1.48 (2H, m), 1.62-1.69 (1H, m), 1.73-1.79 (2H, m), 1.99-2.04 (2H, m), 3.89-3.97 (1H, m), 4.06 (3H, t, J = 1.4 Hz), 5.79-5.82 (1H, m), 7.28-7.35 (2H, m).

### Synthesis Example 11

In the same manner as in Synthesis Example 3, using 3,5-difluoro-4-methoxybenzoyl chloride in place of 3,4-dimethoxybenzoyl chloride, N-((1S)-1-cyclohexylethyl)-3,5-difluoro-4-methoxybenzamide (hereinafter, referred to as the present compound 11) was obtained.

### Present compound 11

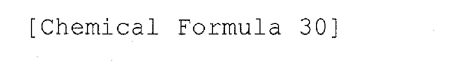

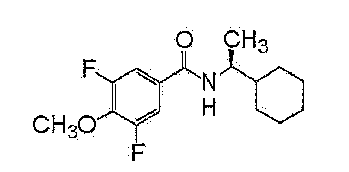

¹H-NMR (CDCl₃) δ: 0.97-1.29 (8H, m), 1.38-1.46 (1H, m), 1.66-1.79 (5H, m), 4.01-4.08 (4H, m), 5.76 (1H, d, J = 8.7 Hz), 7.29-7.35 (2H, m).

### Synthesis Example 12

In the same manner as in Synthesis Example 3, using cyclohexylamine in place of (1S)-1-cyclohexylethylamine and using 3-fluoro-4-methoxybenzoyl chloride in place of 3,4-dimethoxybenzoyl chloride, N-(cyclohexyl)-3-fluoro-4-methoxybenzamide (hereinafter, referred to as the present compound 12) was obtained.

### Present compound 12

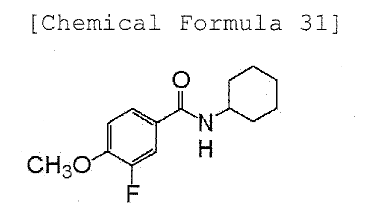

¹H-NMR (CDCl₃) δ: 1.16-1.27 (3H, m), 1.37-1.48 (2H, m), 1.63-1.79 (3H, m), 2.00-2.04 (2H, m), 3.90-3.99 (4H, m), 5.83 (1H, d, J = 6.3 Hz), 6.95-6.99 (1H, m), 7.49-7.52 (2H, m).

### Production Example 13

In the same manner as in Synthesis Example 3, 3-fluoro-4-methoxybenzoyl chloride in place of 3,4-dimethoxybenzoyl chloride, N-((1S)-1-cyclohexylethyl)-3-fluoro-4-methoxybenzamide (hereinafter, referred to as the present compound 13) was obtained.

### Present compound 13

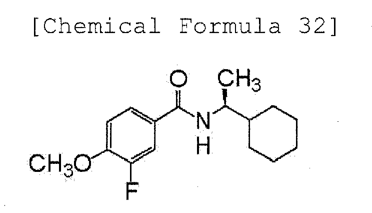

¹H-NMR (CDCl₃) δ: 0.98-1.27 (8H, m), 1.38-1.46 (1H, m), 1.65-1.81 (5H, m), 3.93 (3H, s), 4.00-4.11 (1H, m), 5.79 (1H, d, J = 8.9 Hz), 6.96-7.00 (1H, m), 7.49-7.52 (2H, m).

### Production Example 14

In the same manner as in Synthesis Example 3, using 2-methylcyclohexylamine in place of (1S)-1-cyclohexylethylamine and using 3-fluoro-4-methoxybenzoyl chloride in place of 3,4-dimethoxybenzoyl chloride, N-(2-methylcyclohexyl)-3-fluoro-4-methoxybenzamide (hereinafter, referred to as the present compound 14) was obtained. Present compound 14 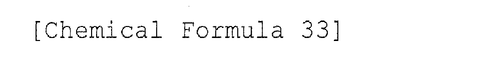 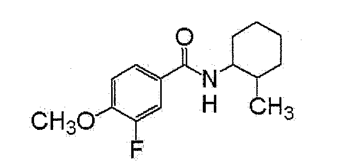 ¹H-NMR (CDCl₃) δ: 0.93 (0.9H, d, J = 7.0 Hz), 0.96 (2.1H, d, J = 6.5 Hz), 1.08-2.01 (9.0H, m), 3.62-3.71 (0.7H, m), 3.92 (2.1H, s), 3.92 (0.9H, s), 4.21-4.26 (0.3H, m), 6.12-6.15 (1.0H, m), 6.93-7.00 (1.0H, m), 7.51-7.57 (2.0H, m).

### Synthesis Example 15

In the same manner as in Synthesis Example 3, 2-methylcyclopentylamine in place of (1S)-1-cyclohexylethylamine and using 3-fluoro-4-methoxybenzoyl chloride in place of 3,4-dimethoxybenzoyl chloride, N-(2-methylcyclopentyl)-3-fluoro-4-methoxybenzamide (hereinafter, referred to as the present compound 15) was obtained.

### Present compound 15

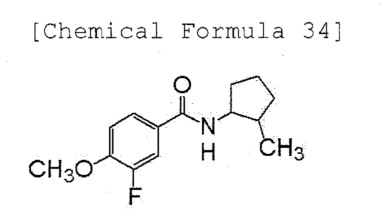

¹H-NMR (CDCl₃) δ: 0.93 (0.9H, d, J = 7.1 Hz), 1.06 (2.1H, d, J = 6.6 Hz), 1.22-2.28 (7.0H, m), 3.91-3.99 (3.7H, m), 4.38-4.45 (0.3H, m), 6.02 (0.3H, d, J = 8.5 Hz), 6.13 (0.7H, d, J = 7.3 Hz), 6.93-6.98 (1.0H, m), 7.50-7.55 (2.0H, m).

### Synthesis Example 16

In the same manner as in Synthesis Example 3, 2-methylcyclopentylamine in place of (1S)-1-cyclohexylethylamine and using 3,5-difluoro-4-methoxybenzoyl chloride in place of 3,4-dimethoxybenzoyl chloride, N-(2-methylcyclopentyl)-3,5-difluoro-4-methoxybenzamide (hereinafter, referred to as the present compound 16) was obtained.

### Present compound 16

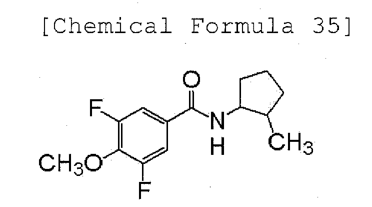

¹H-NMR (CDCl₃) δ: 0.93 (0.9H, d, J = 6.8 Hz), 1.05 (2.1H, d, J = 6.6 Hz), 1.22-2.28 (7.0H, m), 3.88-3.96 (0.7H, m), 4.04-4.05 (3.0H, m), 4.36-4.43 (0.3H, m), 6.13 (0.3H, d, J = 7.8 Hz), 6.30 (0.7H, d, J = 7.6 Hz), 7.28-7.38 (2.0H, m).

### Synthesis Example 17

In the same manner as in Synthesis Example 3, 2-chlorocyclohexylamine hydrochloride in place of (1S)-1-cyclohexylethylamine and using 3,5-difluoro-4-methoxybenzoyl chloride in place of 3,4-dimethoxybenzoyl chloride, N-(2-chlorocyclohexyl)-3,5-difluoro-4-methoxybenzamide (hereinafter, referred to as the present compound 17) was obtained.

### Present compound 17

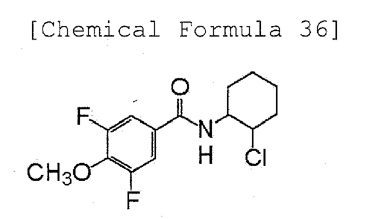

¹H-NMR (CDCl₃) δ: 1.28-1.52 (3H, m), 1.73-1.85 (3H, m), 2.28-2.34 (2H, m), 3.84 (1H, td, J = 10.6, 4.1 Hz), 3.96-4.09 (4H, m), 5.99 (1H, d, J = 7.2 Hz), 7.31-7.38 (2H, m).

### Synthesis Example 18

In the same manner as in Synthesis Example 3, 1-cyclobutylethylamine in place of (1S)-1-cyclohexylethylamine and using 3,5-difluoro-4-methoxybenzoyl chloride in place of 3,4-dimethoxybenzoyl chloride, N-(1-cyclobutylethyl)-3,5-difluoro-4-methoxybenzamide (hereinafter, referred to as the present compound 18) was obtained.

### Present compound 18

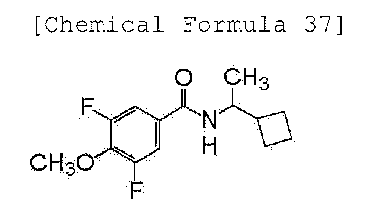

¹H-NMR (CDCl₃) δ: 1.12 (3H, d, J = 6.5 Hz), 1.74-1.90 (4H, m), 1.97-2.07 (2H, m), 2.31-2.37 (1H, m), 4.04-4.17 (4H, m), 5.97 (1H, d, J = 8.2 Hz), 7.29-7.37 (2H, m).

### Synthesis Example 19

In the same manner as in Synthesis Example 3, (1-hydroxycyclohexyl)methylamine hydrochloride in place of (1S)-1-cyclohexylethylamine and using 3,5-difluoro-4-methoxybenzoyl chloride in place of 3,4-dimethoxybenzoyl chloride, N-(1-hydroxycyclohexyl)methyl-3,5-difluoro-4-methoxybenzamide (hereinafter, referred to as the present compound 19) was obtained.

### Present compound 19

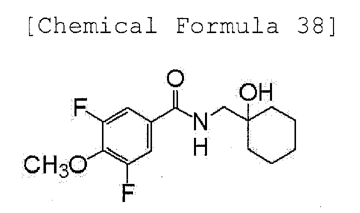

¹H-NMR (CDCl₃) δ: 1.31-1.61 (10H, m), 2.25 (1H, s), 3.47 (2H, d, J = 6.0 Hz), 4.06 (3H, dd, J = 1.4, 1.4 Hz), 6.58-6.61 (1H, m), 7.32-7.40 (2H, m).

### Synthesis Example 20

To 2 ml of ethyl acetate were added 0.30 g of 3,4,5-trimethoxybenzoyl chloride, 0.30 g of 2-methylpropylamine and 0.25 g of triethylamine, followed by stirring at room temperature for 4 hours. The reaction mixture was directly subjected to silica gel column chromatography, and 0.16 g of N-(2-methylpropyl)-3,4,5-trimethoxybenzamide was obtained (hereinafter, referred to as the present compound 20).

### Present compound 20

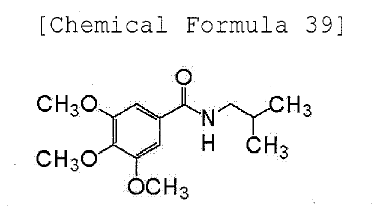

¹H-NMR (CDCl₃) δ: 0.99 (6H, d, J = 6.8 Hz), 1.88-1.94 (1H, m), 3.28 (2H, dd, J = 6.4, 6.4 Hz), 3.88 (3H, s), 3.91 (6H, s), 6.09 (1H, br s), 6.98 (2H, s).

### Synthesis Example 21

To 2 ml of ethyl acetate were added 0.30 g of 3,4-dimethoxybenzoyl chloride, 0.30 g of 2-methylpropylamine and 0.25 g of triethylamine, followed by stirring at room temperature for 4 hours. The reaction mixture was directly subjected to silica gel column chromatography, and 0.36 g of N-(2-methylpropyl)-3,4-dimethoxybenzamide was obtained (hereinafter, referred to as the present compound 21). 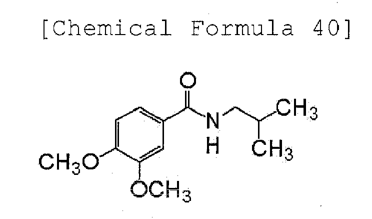 ¹H-NMR (CDCl₃) δ: 0.98 (6H, d, J = 6.5 Hz), 1.87-1.94 (1H, m), 3.28 (2H, dd, J = 6.5, 6.5 Hz), 3.92 (3H, s), 3.94 (3H, s), 6.12 (1H, br s), 6.86 (1H, d, J = 8.2 Hz), 7.25 (1H, dd, J = 8.2, 2.1 Hz), 7.43 (1H, d, J = 2.1 Hz).

### Synthesis Example 22

To 2 ml of ethyl acetate were added 0.20 g of 3,5-difluoro-4-methoxybenzoyl chloride, 0.11 g of 1,2-dimethylpropylamine and 0.20 ml of triethylamine, followed by stirring at room temperature for 5 hours. The reaction mixture was concentrated under reduced pressure. The residue was subjected to silica gel column chromatography, and 0.22 g of N-(1,2-dimethylpropyl)-3,5-difluoro-4-methoxybenzamide was obtained (hereinafter, referred to as the present compound 22).

### Present compound 22

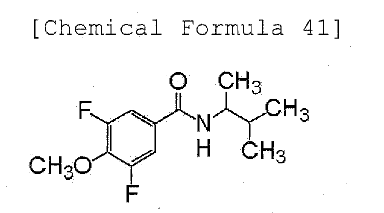

¹H-NMR (CDCl₃) δ: 0.95 (3H, d, J = 4.4 Hz), 0.97 (3H, d, J = 4.1 Hz), 1.18 (3H, d, J = 6.8 Hz), 1.76-1.84 (1H, m), 4.01-4.08 (4H, m), 5.75 (1H, d, J = 7.1 Hz), 7.27-7.35 (2H, m).

### Synthesis Example 23

In the same manner as in Synthesis Example 22, (1S)-1,2-dimethylpropylamine in place of 1,2-dimethylpropylamine, N-((1S)-1,2-dimethylpropyl)-3,5-difluoro-4-methoxybenzamide (hereinafter, referred to as the present compound 23) was obtained.

### Present compound 23

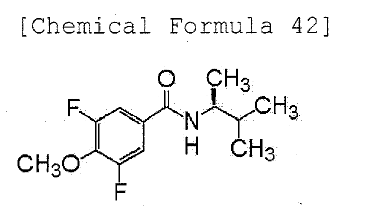

¹H-NMR (CDCl₃) δ: 0.95 (3H, d, J = 4.3 Hz), 0.97 (3H, d, J = 4.3 Hz), 1.18 (3H, d, J = 6.8 Hz), 1.76-1.84 (1H, m), 4.02-4.07 (4H, m), 5.75 (1H, d, J = 8.0 Hz), 7.27-7.34 (2H, m).

### Synthesis Example 24

In the same manner as in Synthesis Example 22, (1S)-1,2,2-trimethylpropylamine in place of 1,2-dimethylpropylamine, N-((1S)-1,2,2-trimethylpropyl)-3,5-dlfluoro-4-methoxybenzamide (hereinafter, referred to as the present compound 24) was obtained.

### Present compound 24

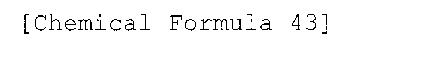

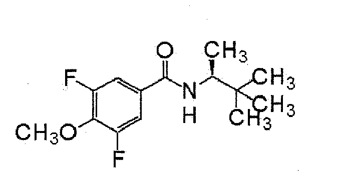

¹H-NMR (CDCl₃) δ: 0.96 (9H, s), 1.15 (3H, d, J = 7.0 Hz), 4.03-4.10 (4H, m), 578 (1H, d, J = 8.5 Hz), 7.27-7.34 (2H, m).

### Synthesis Example 25

In the same manner as in Synthesis Example 20, 1,2-dimethylpropylamine in place of 2-methylpropylamine and using 3-fluoro-4-methoxybenzoyl chloride in place of 3,4,5-trimethoxybenzoyl chloride, N-(1,2-dimethylpropyl)-3-fluoro-4-methoxybenzamide (hereinafter, referred to as the present compound 25) was obtained.

### Present compound 25

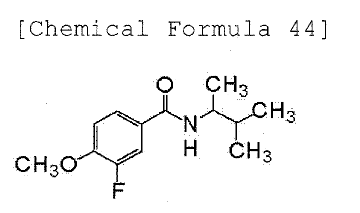

¹H-NMR (CDCl₃) δ: 0.95 (3H, d, J = 4.6 Hz), 0.97 (3H, d, J = 4.6 Hz), 1.18 (3H, d, J = 6.8 Hz), 1.77-1.85 (1H, m), 3.93 (3H, s), 4.01-4.11 (1H, m), 5.78 (1H, d, J = 8.2 Hz), 6.96-7.00 (1H, m), 7.49-7.53 (2H, m).

### Production Example 26

In the same manner as in Synthesis Example 20, (1S)-1,2-dimethylpropylamine in place of 2-methylpropylamine and using 3-fluoro-4-methoxybenzoyl chloride in place of 3,4,5-trimethoxybenzoyl chloride, N-((1S)-1,2-dimethylpropyl)-3-fluoro-4-methoxybenzamide (hereinafter, referred to as the present compound 26) was obtained.

### Present compound 26

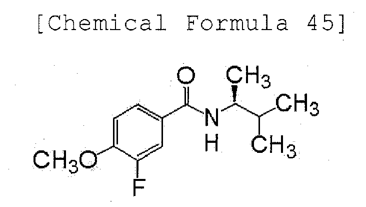

¹H-NMR (CDCl₃) δ: 0.94 (3H, d, J = 5.6 Hz), 0.96 (3H, d, J = 5.6 Hz), 1.16 (3H, d, J = 6.8 Hz), 1.76-1.84 (1H, m), 3.91 (3H, s), 4.00-4.06 (1H, m), 6.19 (1H, d, J = 8.7 Hz), 6.92-6.96 (1H, m), 7.52-7.56 (2H, m).

### Synthesis Example 27

In the same manner as in Synthesis Example 20, (1S)-1,2,2-trimethylpropylamine in place of 2-methylpropylamine and using 3-fluoro-4-methoxybenzoyl chloride in place of 3,4,5-trimethoxybenzoyl chloride, N-((1S)-1,2,2-trimethylpropyl)-3-fluoro-4-methoxybenzamide (hereinafter, referred to as the present compound 27) was obtained. Present compound 27 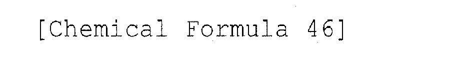 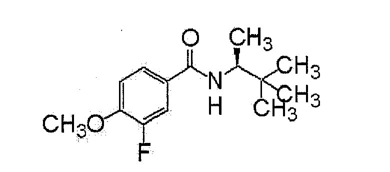 ¹H-NMR (CDCl₃) δ: 0.97 (9H, s), 1.16 (3H, d, J = 6.8 Hz), 3.93 (3H, s), 4.05-4.12 (1H, m), 5.80 (1H, d, J = 10.1 Hz), 6.96-7.00 (1H, m), 7.49-7.52 (2H, m).

### Synthesis Example 28

In the same manner as in Synthesis Example 20, an L-valine methyl ester in place of 2-methylpropylamine, N-((1S)-1-methoxycarbonyl-2-methylpropyl)-3,4,5-trimethoxybenzamide (hereinafter referred to as the present compound 28) was obtained.

### Present compound 28

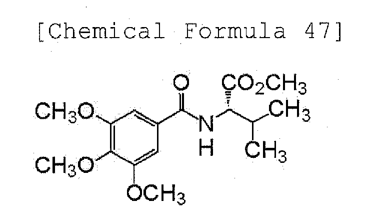

¹H-NMR (CDCl₃) δ: 1.00 (3H, d, J = 6.9 Hz), 1.02 (3H, d, J = 6.9 Hz), 2.22-2.34 (1H, m), 3.79 (3H, s), 3.89 (3H, s), 3.92 (6H, s), 4.77 (1H, dd, J = 8.6, 5.1 Hz), 6.55 (1H, d, J = 8.6 Hz), 7.03 (2H, s).

Next, Reference Synthesis Examples for production of synthetic intermediates of the compound of the present invention are shown.

### Reference Synthesis Example 1

To a solution of 50 g of potassium permanganate and 1,000 ml of water, a solution of 35 g of 3,4,5-trifluorobenzaldehyde and 100 ml of acetone was added dropwise at room temperature over 30 minutes, followed by stirring at room temperature for 6 hours. While cooling the reaction mixture, 65 ml of concentrated hydrochloric acid and 135 g of sodium hydrogen sulfite were added to the reaction mixture, followed by extraction twice with ethyl acetate. The organic layer was washed sequentially with water and brine, dried over magnesium sulfate and then concentrated under reduced pressure. The resultant residue was washed with hexane to obtain 30 g of 3,4,5-trifluorobenzoic acid.

### 3,4,5-Trifluorobenzoic acid

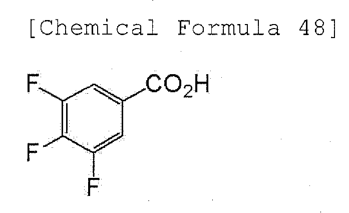

¹H-NMR (CDCl₃) δ: 7.73-7.81 (2H, m), 12.21 (1H, br s).

To 100 ml of toluene, 11 g of 3,4,5-trifluorobenzoic acid and 30 g of a 28% solution of sodium methoxide in methanol were added, and then the mixture was heated under reflux for 6 hours. The reaction mixture cooled to about room temperature was added into water. After washing with MTBE, the aqueous layer was extracted. The aqueous layer was acidified by addition of hydrochloric acid, extracted with ethyl acetate, and then washed sequentially with water and brine. The organic layer was dried over magnesium sulfate and then concentrated under reduced pressure, and6.0 g of 3,5-difluoro-4-methoxybenzoic acid was obtained. 3,5-Difluoro-4-methoxybenzoic acid 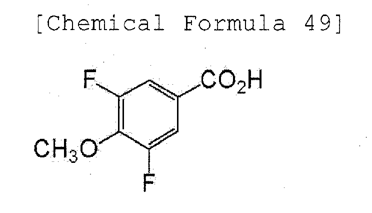 ¹H-NMR (CDCl₃) δ: 4.13 (3H, t, J = 1.7 Hz), 7.61-7.69 (2H, m), 11.47 (1H, br s).

To 50 ml of toluene were added 6.0 g of 3,5-difluoro-4-methoxybenzoic acid and 6.0 g of thionyl chloride. The mixture was heated under reflux for 2 hours. The reaction mixture cooled to about room temperature was concentrated under reduced pressure to obtain 5.3 g of 3,5-difluoro-4-methoxybenzoyl chloride.

### 3,5-Difluoro-4-methoxybenzoyl chloride

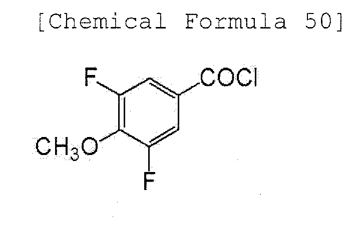

¹H-NMR (CDCl₃) δ: 4.18 (3H, t, J = 2.1 Hz), 7.65-7.73 (2H, m).

Then, Formulation Examples are shown. The term "part(s)" represents part(s) by weight.

### Formulation Example 1

Fifty (50) parts of any one of the present compounds 1 to 28, 3 parts of calcium ligninsulfonate, 2 parts of magnesium laurylsulfate and 45 parts of synthetic hydrated silicon oxide are pulverized and mixed well to give a wettable powder of each compound.

### Formulation Example 2

20 parts of any one of the present compounds 1 to 28 and 1.5 parts of sorbitan trioleate are mixed with 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol is added. The mixture was stirred and mixed to give a flowable of each compound.

### Formulation Example 3

2 parts of any one of the present compounds 1 to 28, 88 parts of kaolin clay and 10 parts of talc are pulverized and mixed well to give a dust of each compound.

### Formulation Example 4

5 parts of each of the present compounds 1 to 28, 14 parts of polyoxyethylenestyryl phenyl ether, 6 parts of calcium dodecylbenzenesulfonate and 75 parts of xylene are mixed well to give an emulsifiable concentrate of each compound.

### Formulation Example 5

2 parts of any one of the present compounds 1 to 28, 1 part of synthetic hydrated silicon oxide, 2 parts of calcium ligninsulfonate, 30 parts of bentonite and 65 parts of kaolin clay are pulverized and mixed well, and water is added thereto and kneeded well, granulated and dried to give granules of each compound.

### Formulation Example 6

10 parts of any one of the present compounds 1 to 28, 35 parts of a polyoxyethylenealkyl ether sulfate ammonium salt/white carbon (mass ratio: 50/50), and 55 parts of water are mixed and wet pulverized finely to give a formulation of each compound.

The following Test Examples show that the plant disease control agent of the present invention is useful for controlling a plant disease. In each Test Example, spraying or treatment was carried out so that one plant was sprayed or treated with one kind of the control agents.

The controlling effect was evaluated by visually observing the area or lesion spots on each of test plants at the time of investigation and comparing the area of lesion spots on a plant treated with the present compound with that on an untreated plant.

### Test Example 1

### Test of preventive effect on wheat powdery mildew (Erysiphe graminis f. sp. tritici)

Each of plastic pots was filled with sandy loam and sown with wheat (cultivar; Shirogane), followed by growing in a greenhouse for 10 days. Each of the present compounds 1 to 16 and 20 to 28 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling. The wheat which was applied by the dilution, the plant was air-dried and then inoculated by sprinkling with spores of *Erysiphe graminis f. sp. tritici*. Separately, the wheat seedling which was not applied by any control agent was similarly inoculated by sprinkling with the spores. After the inoculation, the plant was held in a greenhouse at 23°C for 7 days and the area of lesion spots was investigated. As a result, it was found that the area of lesion spots on the plant treated with the control agent containing any one of the present compounds 4, 8, 10, 22, 23, 26 and 27 was 30% or less of that on the untreated plant.

### Test Example 2

### Test of preventive effect on wheat Glume blotch

### (Stagonospora nodorum)

Each of plastic pots was filled with sandy loam and sown with wheat (cultivar; Shirogane), followed by growing in a greenhouse for 10 days. Each of the present compounds 1 to 16 and 20 to 28 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling. The wheat which was applied by the dilution was air-dried and then inoculated by spraying a water suspension of spores of *Stagonospora nodorum.* Separately, the wheat seedling which was not applied by any control agent was similarly inoculated by spraying of the spores.

After the inoculation, the plant was held under darkness and high humidity conditions at 18°C for 4 days and then under lighting conditions for 4 days, and then the area of lesion spots was investigated. As a result, it was found that the area of lesion spots on the plant treated with any one of the present compounds 7, 10, 22, 23, 24 and 27 was 30% or less of that on the untreated plant.

### Test Example 3

### Test of preventive effect on wheat Fusarium blight

### (Fusarium culmorum)

Each of plastic pots was filled with sandy loam and sown with wheat (cultivar; Shirogane), followed by growing in a greenhouse for 10 days. Each of the present compounds 1 to 16 and 20 to 28 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown wheat seedling. The wheat which was applied by the dilution was air-dried and then inoculated by spraying a water suspension of spores of *Fusarium culmorum.* Separately, the wheat seedling which was not applied by any control agent was similarly inoculated by spraying of the spores. After the inoculation, the plant was held under darkness and high humidity conditions at 23°C for 4 days and then under lighting conditions for 3 days, and then the area oflesion spots was investigated. As a result, it was found that the area of lesion spots on the plant treated with the control agent containing any one of the present compounds 7, 8, 11 and 27 was 30% or less of that on the untreated plant.

### Test Example 4

### Test of preventive effect on cucumber gray mold (Botrytis cinerea)

Each of plastic pots was filled with sandy loam and sown with cucumber (variety; Sagamihanjiro), followed by growing in a greenhouse for 12 days. Each of the present compounds 1 to 28 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown cucumber seedling. The cucumber which was applied by the dilution was air-dried and a PDA medium containing spores of Botrytis cinerea was placed on the surfaces of the cucumber leaves. Separately, the cucumber seedling which was not applied by any control agent was similarly inoculated with the spores. After the inoculation, the plant was grown at 12°C and high humidity for 4 days. Then, the area of lesion spots was investigated. As a result, it was found that the area of lesion spots on the plant treated with the control agent containing the present compounds 4, 8, 10, 17, 18, 22 and 23 was 30% or less of that on the untreated plant.

### Test Example 5

### Test of preventive effect on cucumber stem rot (Sclerotinia sclerotiorum)

Each of plastic pots was filled with sandy loam and sown with cucumber (variety; Sagamihanjiro), followed by growing in a greenhouse for 12 days. Each of the present compounds 7 to 19 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown cucumber seedling. The cucumber which was applied by the dilution was air-dried and a PDA medium containing mycelia of Sclerotinia sclerotiorum was placed on the surfaces of the cucumber leaves. Separately, the cucumber seedling which was not applied by any control agent was similarly inoculated with the mycelia. After the inoculation, the plant was grown at 18°C and high humidity for 4 days. Then, the area of lesion spots was investigated. As a result, it was found that the area of lesion spots on the plant treated with the control agent containing any one of the present compounds 8, 9, 10, 17, 18 and 19 was 30% or less of that on the untreated plant.

### Test Example 6

### Test of preventive effect on Alternaria leaf spot

### (Alternaria brassicicola)

Each of plastic pots was filled with sandy loam and sown with Japanese radish (cultivar: Wase 40-nichi), followed by growing in a greenhouse for 5 days. Each of the present compounds 1 to 19 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown Japanese radish seedling. After the foliage application, the plant was air-dried and then inoculated by spraying a water suspension of spores of Alternaria brassicicola. Separately, the Japanese radish seedling which was not applied by any control agent was similarly inoculated by spraying of the spores. After the inoculation, the plant was held under high humidity conditions at 24°C for 1 day and then in a greenhouse for 3 days, and then the area oflesion spots was investigated. As a result, it was found that the area of lesion spots on the plant treated with the control agent containing the present compound 4 or 19 was 30% or less of that on the untreated plant.

### Test Example 7

### Test of preventive effect on grape Downy mildew (Plasmopara viticola)

Each of plastic pots was filled with sandy loam and sown with grape (cultivar: seedling of Berry-A), followed by growing in a greenhouse for 40 days. The grown grape seedling was inoculated by spraying a water suspension of zoosporangia of Plasmopara viticola. After that, the plant was held under high humidity conditions at 23°C for 1 day and then air-dried to give a Plasmopara viticola-infected seedling. Each of the present compounds 10 to 19 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grape seedling. After the foliage application, the plant was air-dried, and held in a greenhouse at 23°C for 5 days and then under high humidity conditions at 23°C for 1 day. Then, the area of lesion spots was investigated. As a result, it was found that the area of lesion spots on the plant treated with the control agent containing the present compound 18 or 19 was 30% or less of that the untreated plant.

### Test Example 8

### Test of preventive effect on tomato late blight

### (Phytophthora infestans)

Each of plastic pots was filled with sandy loam and sown with tomato (cultivar; Patio), followed by growing in a greenhouse for 20 days. Each of the present compounds 1 to 28 was formulated into a flowable formulation according to Formulation Example 6. The flowable formulation was diluted to a predetermined concentration (500 ppm) with water, and foliage application of the dilution was carried out so that the dilution could adhere sufficiently to the surfaces of leaves of the grown tomato seedling. After the plant was air-dried so that the diluted solution on the leaf surfaces was dried, a water suspension of spores of *Phytophthora infestans* was sprayed. After the inoculation, the plant was held under high humidity conditions at 23°C for 1 day and held in a greenhouse for 4 days, and then the area of lesion spots was investigated. As a result, it was found that the area of lesion spots on the plant treated with the control agent containing any one of the present compounds 1, 2, 3, 5 to 11, 15 to 17, 18, 22 to 26 and 27 was 30% or less of that an untreated plant.

### Test Example 9

### Test of preventive effect on tomato late blight

### (Phytophthora infestans)

Each of plastic pots was filled with sandy loam and sown with tomato (cultivar; Patio), followed by growing in a greenhouse for 20 days. Each of the present compounds 7 and 9 was formulated into a flowable formulation according to Formulation Example 6. The flowable formulation was diluted to a compound concentration of 200 ppm or 50 ppm with water to prepare a test solution. Foliage application of the test solution was carried out so that the test solution could adhere sufficiently to the surfaces of leaves of the grown tomato seedling. After the plant was air-dried so that the diluted solution on the leaf surfaces was dried, a water suspension of zoosporangia of *Phytaphthora infestans* (about 30,000 zoosporangia/ml) was sprayed (about 2 ml of the suspension per a plant). After the inoculation, the tomato seedling was held under relative humidity 90% or more conditions at 23°C for 1 day and then in a greenhouse at 24°C during day and at 20°C at night for 4 days. Then, the area of lesion spots of late blight on the tomato seedling was investigated. Results are shown in Table 1.

**[Table 1]**

| | Concentration of present compound [ppm] | Control value |
|---|---|---|
| Present compound 7 | 200 | ++++ |
| | 50 | ++ |
| Present compound 9 | 200 | ++++ |
| | 50 | ++++ |

| | | |
|---|---|---|
| Control value is as follows. -: 80% or more of the lesion spot area on an untreated plant. +: 60% or more and less than 80% of the lesion spot area on an untreated plant. ++: 40% or more and less than 60% of the lesion spot area on an untreated plant. +++: 20% or more and less than 40% of the lesion spot area on an untreated plant. ++++: Less than 20% of the lesion spot area on an untreated plant. | | |

### Industrial Applicability

The plant disease control agent of the present invention has excellent plant disease controlling activity and therefore it is useful.

## Claims

1. A plant disease control agent comprising, as an active ingredient, an amide compound represented by formula (1): 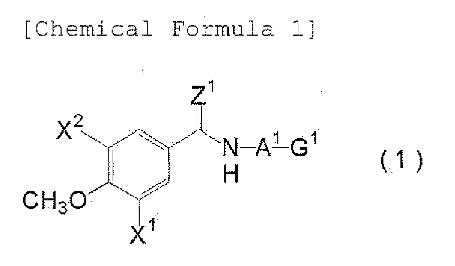 wherein
X¹ represents a fluorine atom or a methoxy group,
X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group or a CONR⁴R⁵ group, or a phenyl group optionally substituted with at least one member selected from the group consisting of a methyl group, a halogen atom, a cyano group and a nitro group,
Z¹ represents an oxygen atom or a sulfur atom,
A¹ represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G¹ represents a CR⁶R⁷R⁸ group (provided that the aforementioned A¹ is not a single bond when G¹ is a CR⁶R⁷R⁸ group), a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ represents a C1-C4 alkyl group, a C3-C4 alkenyl group or a C3-C4 alkynyl group,
R⁴ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a hydrogen atom, a C1-C4 alkyl group, a C3-C4 alkenyl group, a C3-C4 alkynyl group or a C2-C4 haloalkyl group,
R⁶ and R⁷ independently represent a C1-C4 alkyl group, R⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR⁹R¹⁰ group, in which R⁹ and R¹⁰ are as defined above.

2. The plant disease control agent according to claim 1, wherein in the formula (1),
A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming the ring is replaced by a carbonyl group, or a C3-C6 hydroxyiminocycloalkyl group optionally substituted with at least one member selected from the group [a-1].

3. The plant disease control agent according to claim 2, wherein in the formula (1),
X² is a hydrogen atom or a halogen atom, and
G¹ is a C3-C6 cycloalkyl group optionally substituted with at least one member selected from the group consisting of a halogen atom, a C1-C4 alkyl group and a hydroxyl group.

4. The plant disease control agent according to claim 1, wherein in the formula (1),
A¹ is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a CR⁶R⁷R⁸ group.

5. The plant disease control agent according to claim 4, wherein in the formula (1),
X² is a hydrogen atom or a halogen atom,
A¹ is -CH₂-, -CH(CH₃)-, or methylene substituted with a C2-C5 alkoxycarbonyl group, and
R⁸ is a hydrogen atom or a C1-C4 alkyl group.

6. A plant disease control method which comprises applying an effective amount of an amide compound represented by the formula (1) to plants or soil: 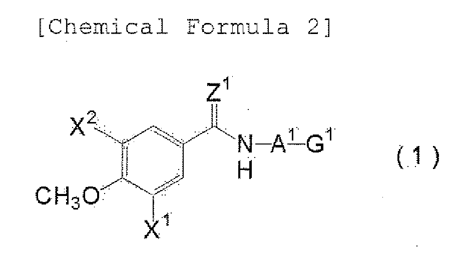 wherein
X¹ represents a fluorine atom or a methoxy group,
X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group or a CONR⁴R⁵ group, or a phenyl group optionally substituted with at least one member selected from the group consisting of a methyl group, a halogen atom, a cyano group and a nitro group,
Z¹ represents an oxygen atom or a sulfur atom, A¹ represents a single bond, -CH₂-, -CH(CH₃)-, -C(CP₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G¹ represents a CR⁶R⁷R⁸ group (provided that the aforementioned A¹ is not a single bond when G¹ is a CR⁶R⁷R⁸ group), a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ represents a C1-C4 alkyl group, a C3-C4 alkenyl group or a C3-C4 alkynyl group,
R⁴ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a hydrogen atom, a C1-C4 alkyl group, a C3-C4 alkenyl group, a C3-C4 alkynyl group or a C2-C4 haloalkyl group,
R⁶ and R⁷ independently represent a C1-C4 alkyl group,
R⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR⁹R¹⁰ group, in which R⁹ and R¹⁰ are as defined above.

7. The plant disease control method according to claim 6, wherein in the formula (1),
A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming ring is replaced by a carbonyl group, or a C3-C6 hydroxyiminocycloalkyl group optionally substituted with at least one member selected from the group [a-1].

8. The plant disease control method according to claim 7, wherein in the formula (1),
X² is a hydrogen atom or a halogen atom, and
G¹ is a C3-C6 cycloalkyl group optionally substituted with at least one member selected from the group consisting of a halogen atom, a C1-C4 alkyl group and a hydroxyl group.

9. The plant disease control method according to claim 6, wherein in the formula (1),
A¹ is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a CR⁶R⁷R⁸ group.

10. The plant disease control method according to claim 9, wherein in the formula (1),
X² is a hydrogen atom or a halogen atom,
A¹ is -CH₂-, -CH(CH₃)-, or methylene substituted with a C2-C5 alkoxycarbonyl group, and
R⁸ is a hydrogen atom or a C1-C4 alkyl group.

11. Use of an amide compound represented by the formula (1): 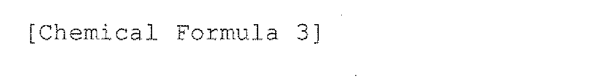 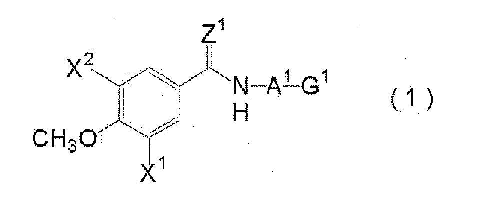 wherein
X¹ represents a fluorine atom or a methoxy group,
X² represents a hydrogen atom, a halogen atom, a C1-C4 alkyl group, a C2-C4 alkenyl group, a C2-C4 alkynyl group, a C1-C4 haloalkyl group, a C1-C4 alkoxy group, a C1-C4 alkylthio group, a C1-C4 hydroxyalkyl group, a nitro group, a cyano group, a formyl group, an NR¹R² group, a CO₂R³ group or a CONR⁴R⁵ group, or a phenyl group optionally substituted with at least one member selected from the group consisting of a methyl group, a halogen atom, a cyano group and a nitro group,
Z¹ represents an oxygen atom or a sulfur atom,
A¹ represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G¹ represents a CP⁶R⁷R⁸ group (provided that the aforementioned A¹ is not a single bond when G¹ is a CR⁶R⁷R⁸ group), a C3-C6 cycloalkyl group optionally substituted with at least one member selected from the group [a-1] shown below, a C3-C6 cycloalkenyl group optionally substituted with at least one member selected from the group [a-1] shown below, a C3-C6 cycloalkyl group optionally substituted with at least one member selected from the group [a-1] shown below wherein one methylene forming ring is replaced by a carbonyl group, or a C3-C6 hydroxyiminocycloalkyl group optionally substituted with at least one member selected from the group [a-1] shown below,
R¹ and R² independently represent a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R³ represents a C1-C4 alkyl group, a C3-C4 alkenyl group or a C3-C4 alkynyl group,
R⁴ represents a hydrogen atom, a C1-C4 alkyl group, a C3-C4 alkenyl group, a C3-C4 alkynyl group, a C2-C4 haloalkyl group, a C2-C5 alkylcarbonyl group, a C2-C5 alkoxycarbonyl group or a C1-C4 alkylsulfonyl group,
R⁵ represents a hydrogen atom, a C1-C4 alkyl group, a C3-C4 alkenyl group, a C3-C4 alkynyl group or a C2-C4 haloalkyl group,
R⁶ and R⁷ independently represent a C1-C4 alkyl group, R⁸ represents a hydrogen atom, a C1-C4 alkyl group, a C2-C4 alkenyl group, a C2-C4 alkynyl group, a cyano group,
a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group or an NR⁹R¹⁰ group, in which R⁹ and R¹⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-1]:
a halogen atom, a C1-C4 alkyl group, a C2-C4 alkenyl group, a C2-C4 alkynyl group, a hydroxyl group, a cyano group, carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group and an NR⁹R¹⁰ group, in which R⁹ and R¹⁰ are as defined above, for controlling plant diseases by applying it to plants or soil.

12. The use of an amide compound according to claim 11, wherein in the formula (1),
A¹ is a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a C3-C6 cycloalkyl group optionally substituted with at least one member selected from the group [a-1], a C3-C6 cycloalkenyl group optionally substituted with at least one member selected from the group [a-1], a C3-C6 cycloalkyl group optionally substituted with at least one member selected from the group [a-1] wherein one methylene forming ring is replaced by a carbonyl group, or a C3-C6 hydroxyiminocycloalkyl group optionally substituted with at least one member selected from the group [a-1].

13. The use of an amide compound according to claim 12, wherein in the formula (1),
X² is a hydrogen atom or a halogen atom, and
G¹ is a C3-C6 cycloalkyl group optionally substituted with at least one member selected from the group consisting of a halogen atom, a C1-C4 alkyl group and a hydroxyl group.

14. The use of an amide compound according to claim 11, wherein in the formula (1),
A¹ is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G¹ is a CR⁶R⁷R⁸ group.

15. The use of an amide compound according to claim 14, wherein in the formula (1),
X² is a hydrogen atom or a halogen atom,
A¹ is -CH₂-, -CH(CH₃)-, or methylene substituted with a C2-C5 alkoxycarbonyl group, and
R⁸ is a hydrogen atom or a C1-C4 alkyl group.

16. A 3,5-difluoro-4-methoxybenzamide compound represented by the formula (A): 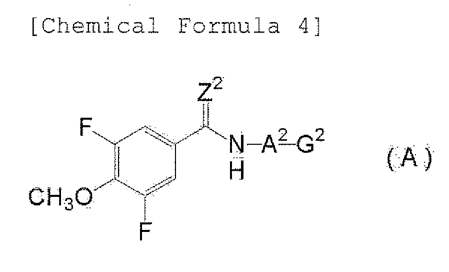 wherein
Z² represents an oxygen atom or a sulfur atom,
A² represents a single bond, -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group,
G² is a CR²⁶R²⁷R²⁸ group (provided that the aforementioned A² is not a single bond when G² is a CR²⁶R²⁷R²⁸ group), a C3-C6 cycloalkyl group optionally substituted with at least one member selected from the group [a-2] shown below, a C3-C6 cycloalkenyl group optionally substituted with at least one member selected from the group [a-2] shown below, a C3-C6 cycloalkyl group optionally substituted with at least one member selected from the group [a-2] shown below wherein one methylene forming ring is replaced by a carbonyl group, or a C3-C6 hydroxyiminocycloalkyl group optionally substituted with at least one member selected from the group [a-2] shown below,
R²⁶ and R²⁷ independently represent a C1-C4 alkyl group,
R²⁸ represents a hydrogen atom, a C1-C4 alkyl group, a C2-C4 alkenyl group, a C2-C4 alkynyl group, a cyano group, a carboxyl group, a C2-C5 alkoxycarbonyl group, a halogen atom, a hydroxyl group, a C1-C6 alkoxy group, a C3-C6 alkenyloxy group, a C1-C6 haloalkyl group, a C2-C6 haloalkoxy group, a C1-C3 alkylthio group, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a C2-C6 cyanoalkyl group, a C1-C3 alkylsulfonyl group, or an NR²⁹R³⁰ group, in which R²⁹ and R³⁰ independently represent a hydrogen atom, a C1-C4 alkyl group, a C2-C5 alkylcarbonyl group, a C2-C5 alkoxycarbonyl group or a C1-C4 alkylsulfonyl group, and
the group [a-2]:
a halogen atom, a C1-C4 alkyl group, a C2-C4 alkenyl group, a C2-C4 alkynyl group, a hydroxyl group, a cyano group, a carboxyl group, a C2-C5 alkoxycarbonyl group, a C1-C6 alkoxy group, a C3-C6 alkenyloxy group, a C1-C6 haloalkyl group, a C1-C6 haloalkoxy group, a phenyl group, a benzyl group, a C1-C3 alkylthio group, a C1-C3 alkylidene group which forms a double bond with a ring-forming carbon atom, a C1-C6 hydroxyalkyl group, a C2-C4 alkylcarbonyloxy group, a (C1-C3 alkylamino)C1-C6 alkyl group, a (di(C1-C3 alkyl)amino)C1-C6 alkyl group, a mercapto group, a carbamoyl group, a formyl group, a C2-C6 cyanoalkyl group, a C1-C3 alkylsulfonyl group, a phenoxy group, and an NR²⁹R³⁰ group, in which R²⁹ and R³⁰ are as defined above.

17. The 3,5-difluoro-4-methoxybenzamide compound according to claim 16, wherein in the formula (A), G² is a C3-C6 cycloalkyl group optionally substituted with at least one member selected from the group [a-2], a C3-C6 cycloalkenyl group optionally substituted with at least one member selected from the group [a-2], a C3-C6 cycloalkyl group optionally substituted with at least one member selected from the group [a-2] wherein one methylene forming ring is replaced by a carbonyl group, or a C3-C6 hydroxyiminocycloalkyl group optionally substituted with at least one member selected from the group [a-2].

18. The 3,5-difluoro-4-methoxybenzamide compound according to claim 17, wherein G² is a C3-C6 cycloalkyl group optionally substituted with at least one member selected from the group consisting of a halogen atom, a C1-C4 alkyl group and a hydroxyl group.

19. A 3,5-difluoro-4-methoxybenzamide compound wherein in the formula (A),
A² is -CH₂-, -CH(CH₃)-, -C(CH₃)₂-, -CH(CH₂CH₃)-, or methylene substituted with at least one member selected from the group consisting of a C1-C3 haloalkyl group, a C2-C4 alkenyl group, a C2-C4 alkynyl group, a cyano group and a C2-C5 alkoxycarbonyl group, and
G² is a CR²⁶R²⁷R²⁸ group.

20. The 3,5-difluoro-4-methoxybenzamide compound according to claim 19, wherein R²⁸ is a hydrogen atom or a C1-C4 alkyl group.

21. The 3,5-difluoro-4-methoxybenzamide compound according to claim 19 or 20, wherein Z² is an oxygen atom and A² is -CH(CH₃)-.
